# EUROPEAN PATENT SPECIFICATION

(11) **EP 3 532 490 B1**
(45) Date of publication and mention of the grant of the patent: **04.08.2021**
(21) Application number: 17797555.4
(22) Date of filing: 27.10.2017
(51) Int. Cl.: C07K 14/725, A61K 31/4188, C07K 16/28

(54) **ADAPTER CHIMERIC ANTIGEN RECEPTOR EXPRESSING CELLS FOR TARGETING OF MULTIPLE ANTIGENS**
ZELLEN MIT EXPRESSION VON CHIMÄREM ADAPTERANTIGENREZEPTOR ZUM TARGETING VON MEHREREN ANTIGENEN
CELLULES EXPRIMANT LE RÉCEPTEUR D'ANTIGÈNE CHIMÉRIQUE D'ADAPTATEUR POUR LE CIBLAGE D'ANTIGÈNES MULTIPLES

(30) Priority: 29.10.2016 EP 16196487
(43) Date of publication of application: 04.09.2019
(73) Proprietor: Miltenyi Biotec B.V. & Co. KG, 51429 Bergisch Gladbach (DE)
(72) Inventor: KAISER, Andrew, 51503 Rösrath (DE); MITTELSTAET, Joerg, 51105 Köln (DE); HUPPERT,Volker, 51515 Kürten (DE); MILTENYI, Stefan, 51429 Bergisch Gladbach (DE); SCHLEGEL, Patrick, 72076 Tübingen (DE); SEITZ, Christian, 72076 Tübingen (DE); LANG, Peter, 72076 Tübingen (DE); HANDGRETINGER, Rupert, 72076 Tübingen (DE)
(74) Representative: Biervert, Christian
(86) International application number: PCT/EP2017/077545
(87) International publication number: WO 2018/078066

(56) References cited:
- WO-A1-2014/100615
- WO-A1-2015/057852
- WO-A1-2016/168769

## Description

### Field of the invention

The present invention generally relates to the field of chimeric antigen receptors (CARs) expressed on immune cells for treatment of cancer, in particular to a CAR that is adaptable to different antigens of target cells.

### Background of the invention

The redirection of T-cells to tumor antigens by expressing transgenic chimeric antigen receptors takes advantage of potent cellular effector mechanisms via human leukocyte antigen (HLA)-independent recognition. The potential of this approach has recently been demonstrated in clinical trials, wherein T-cells expressing CAR were infused into adult and pediatric patients with B-cell malignancies, neuroblastoma, and sarcoma. CARs are recombinant receptors that typically target surface molecules. CARs are typically composed of an extracellular antigen-recognition moiety that is linked, via spacer/hinge and transmembrane domains, to an intracellular signaling domain that can include costimulatory domains and T-cell activation moieties. CARs recognize unprocessed antigens independently of their expression of major histocompatibility antigens, which is unlike the physiologic T-cell receptors (TCRs). Hence, CAR T-cells can circumvent some of the major mechanisms by which tumors avoid major histocompatibility class (MHC)-restricted T-cell recognition such as the downregulation of HLA expression or proteasomal antigen processing, two mechanisms that contribute to tumor escape from TCR-mediated immunity. Another feature of CARs is their ability to bind not only to proteins but also to carbohydrate, ganglioside, proteoglycan, and heavily glycosylated protein, thereby expanding the range of potential targets. CARs engage the target via the antigen recognition moiety, often a single-chain variable fragment (scFv) derived from antibodies or a Fab fragment (reviewed e.g. in Dai et al., 2016, J Natl Cancer Inst 108(7): djv439).

"Universal" CAR systems allow for addressing different antigens using the same CAR.

In WO2015058018A1 the CAR binding motif expressed at the cell surface binds a desired target binding molecule in a non-specific manner, where the binding motif of the CAR is CD16 which is a low affinity Fc receptor that binds unspecifically to Immunoglobulin G antibodies. It is a disadvantage of this system that antibodies in vivo act as natural competitors to the administered antibody of interest supposed to enable specific tumor target lysis upon binding by the CAR T cells.

In WO2012082841A2 a universal, yet adaptable, anti-tag chimeric antigen receptor (AT-CAR) system is disclosed which provides T cells with the ability and specificity to recognize and kill target cells, such as tumor cells, that have been marked by tagged antibodies, e.g. FITC- or biotin labeled antibodies. Here, the disadvantage is that the labels are for the subject to be treated either foreign molecules, e.g. FITC, and therefore immunogenic or the labels are identical to endogenous molecules, e.g. biotin, resulting in blocking of the CAR binding motif by the endogenous molecule which prohibits the CAR from binding its intended molecule-labelled antibody, e.g. biotinylated antibody.

The same universal CAR system is disclosed in WO2013044225A1 facing the same disadvantages.

In WO2014100615A1 a CAR system is disclosed that makes use of CARs that target a moiety that is not produced or expressed by cells of the subject being treated. This CAR system thus allows for focused targeting of the cytotoxic lymphocytes to target cells, such as cancer cells. The targeted moiety is part of a small conjugate molecule (SCM) that also comprises a ligand of a tumor cell receptor. Because small organic molecules are typically used as the targeted moiety, clearance of the SCM from the bloodstream can be achieved within about 20 minutes. By administration of a SCM along with the CAR-expressing cytotoxic lymphocytes, the lymphocyte response can be targeted to only those cells expressing the tumor receptor, thereby reducing off-target toxicity, and the activation of lymphocytes can be more easily controlled due to the rapid clearance of the SCM. The CAR-expressing lymphocytes can also be used as a "universal" cytotoxic cell to target a wide variety of tumors without the need to prepare separate CAR constructs. As the target moieties can be e.g. FITC or biotin, this system faces the same disadvantages as WO2012082841A2 and/or WO2013044225A1.

In WO2015057852A1 switches are disclosed for regulating the activity of a chimeric antigen receptor effector cells (CAR-ECs). The switches generally comprise a chimeric antigen receptor-interacting domain (CAR-ID) and a target interacting domain (TID). The CAR-ID may be e.g. FITC or biotin.

Again, this system faces the same disadvantages as WO2012082841A2 and/or WO2013044225A1.

WO2016168769A1 discloses a CAR T-cell switch, whereby the TCBM comprises an antibody (anti-Her2) linked to a CAR interacting domain via a linker. The label on the TCBM used is FITC, and biotin is suggested as an alternative. Again, this system faces the same disadvantages as WO2012082841A2 and/or WO2013044225A1.

In WO2015057834A1 a chimeric antigen receptor-effector cell switch is disclosed comprising: a) a peptidic antigen that binds a chimeric antigen receptor on an effector cell; and b) a targeting moiety that binds a cell surface molecule on a target cell. The peptidic antigen is based on or derived from a naturally occurring peptide or from a non-naturally occurring peptide.

The disadvantage of this system is that there is a high risk of autoimmunity by tags derived from a human nuclear protein.

In WO2016030414A1 a universal chimeric antigen receptor is disclosed, wherein the receptor comprises three domains, wherein the first domain is a tag-binding domain, the second domain is a linking peptide chain including an extracellular hinge and a transmembrane domain and the third domain is a signal transduction domain, wherein the tag-binding domain binds to a tag derived from any human nuclear protein. In particular suitable tags are peptide sequences from nuclear antigens, which cannot be accessed and bound by the corresponding tag-binding domain in the context of the native protein under physiological conditions. A disadvantage of this system is that the tag can only be added in a co-translational manner, thereby excluding the use of established monoclonal antibodies as tagged antigen binding molecules without prior redesign of the gene encoding said antigen binding molecule. Therefore, there is a need in the art for an improved or alternative universal (adaptable) CAR system.

### Summary of the invention

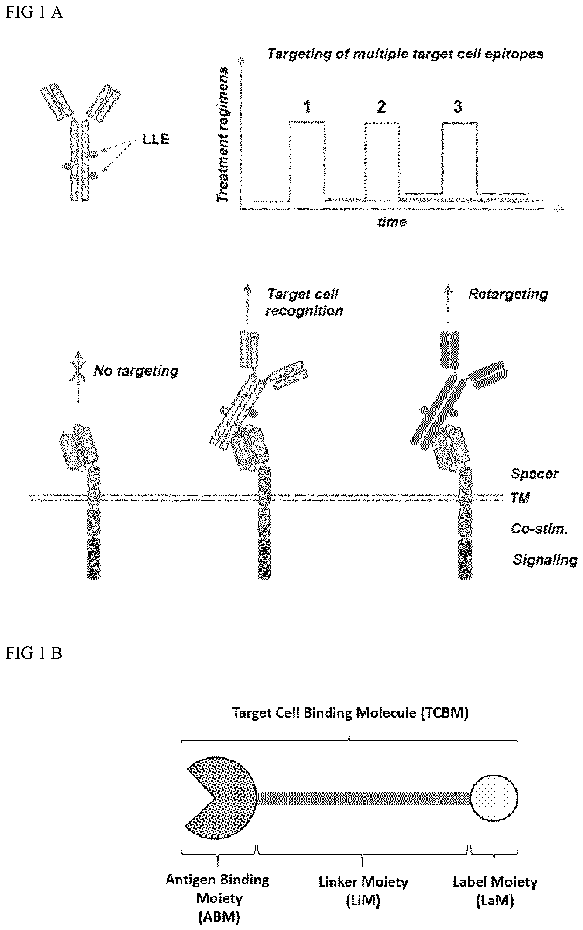

The present invention discloses an approach to control the functions of a cell expressing a chimeric antigen receptor (CAR), e.g.the cytotoxic and/or immunoregulatory functions of an immune cell expressing a chimeric antigen receptor. The adaptable CAR system as disclosed herein comprises a CAR comprising an extracellular binding domain capable of discriminating between a naturally occurring molecule in a subject, wherein said naturally occurring molecule is in the circulatory system of said subject, and a target cell binding molecule (TCBM) comprising an antigen binding molecule (ABM), a label moiety (LaM) and a linker moiety (LiM), wherein said LiM conjugates said ABM and said LaM (see FIG 1B). Said LiM is selected from the group consisting of peptides, alkyl chains, alkanoic acids, carboxylic acids, farnesyls, polyethylene glycols, or a derivative thereof. The label moiety (LaM) is said naturally occurring molecule in a subject or a derivative thereof. The linker moiety (LiM) is coupled to the label moiety (LaM). The chimeric antigen receptor binds TCBMs with LaM and a certain LiM with higher affinity, i.e. an at least 10-fold higher affinity than the endogenous label moiety without linker moiety. Thereby having an improved recognition/binding of TCBMs under physiological conditions where the endogenous LaM might be present.

The benefit of this approach is that the LaM that allows for an adaptable CAR system is non-immunogenic as it is a naturally occurring molecule endogenous to the subject. The LaM is a self antigen, the LaM coupled to the LiM is a modified self antigen, which build a novel epitope, the linker/label epitope (LLE), and the LLE is better bound by the LLE binding domain of the CAR as disclosed herein than the natural occurring molecule in the subject.

Using a pure self antigen as LaM bears a higher risk of an autoimmunity triggered by the immune cell expressing a CAR having an antigen binding domain against the LaM which is a self antigen than using a modified self antigen, the LLE as disclosed herein. The risk of autoimmunity is reduced in the CAR system as disclosed herein as the CAR binds with a lower preference, e.g. with a lower affinity, to the self antigen than to the modified self antigen.

The CAR system as disclosed herein still allows for efficient recruitment of the CAR immune cells to the TCBM that binds specifically to an antigen expressed on a target cell, e.g. a cancer cell. The naturally occurring molecule is a molecule present in the circulatory system of a subject, but is bound at a lower affinity than the TCBM by the CAR as disclosed herein. Preferentially, the naturally occurring molecule in a subject may be an extracellular molecule or a molecule with partial extracellular structure.. Thereby, the present invention improves efficacy of the therapy while minimizing the aforementioned risks of using immunogenic LaM/LiM.

Exemplary the CAR system disclosed herein is shown with a biotin as the label moiety that is a naturally occurring molecule in a subject, with a 6-(6-aminohexanamido) hexanoyl linker moiety (LiM), and with an anti-linker/label epitope (anti-LLE) CAR that binds with at least 10-fold higher preference to the 6-[6-(biotinamido)hexanamido] hexanoyl moiety than to endogenous biotin. The ABM of the TCBM may be any ABM that can bind an antigen of interest on a target cell, e.g. a tumor associated antigen. But no restriction of the invention to this example is intended as the concept can be transferred to other anti-LLE CARs binding with at least 10-fold higher preference to another naturally occurring label moiety coupled with a linker moiety than to the naturally occurring molecule (label moiety) that is not coupled to said linker moiety, wherein said naturally occurring molecule is in the circulatory system of said subject, and wherein said LiM is selected from the group consisting of peptides, alkyl chains, alkanoic acids, carboxylic acids, farnesyls, polyethylene glycols, or a derivative thereof.

The present invention discloses a composition comprising said TCBM and said anti-LLE CAR.

### Brief description of the drawings

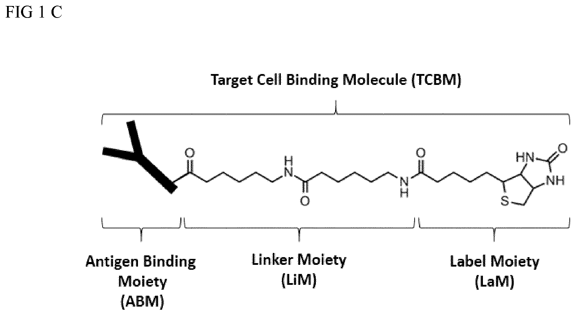

FIG 1: (A) Adapter CAR concept (B) Representation of TCBM composed of antigen binding moiety (ABM), linker moiety (LiM) and label moiety (LaM). (C) Example of (B) comprising the 6-(6-aminohexanamido) hexanoyl linker moiety (LiM) and the biotin label moiety (LaM) and an undefined ABM.

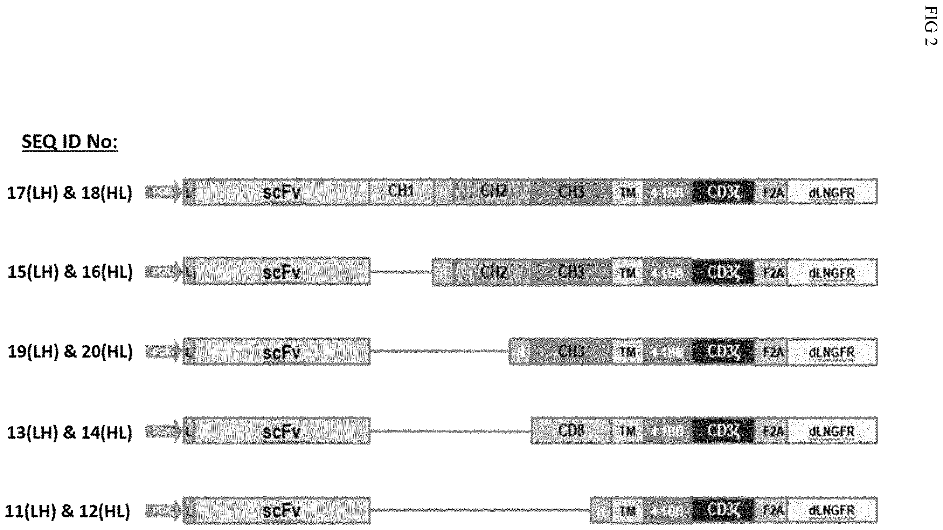

FIG 2: Schematic representation of the different CAR formats tested (SEQ ID NO:11-20). The leader sequence "L" is derived from human CD8, scFvs are derived from SEQ ID NO:1 and SEQ ID NO:2, hinge and spacers "H" and "CH1", "CH2", "CH3" and "CD8" are derived from human IgG4 or human CD8, respectively. The transmembrane domain "TM" is from human CD8, while co-stimulatory and signaling domains are from 4-1 BB and CD3z. All CARs are expressed together with the marker gene LNGFR linked by a 2A element.

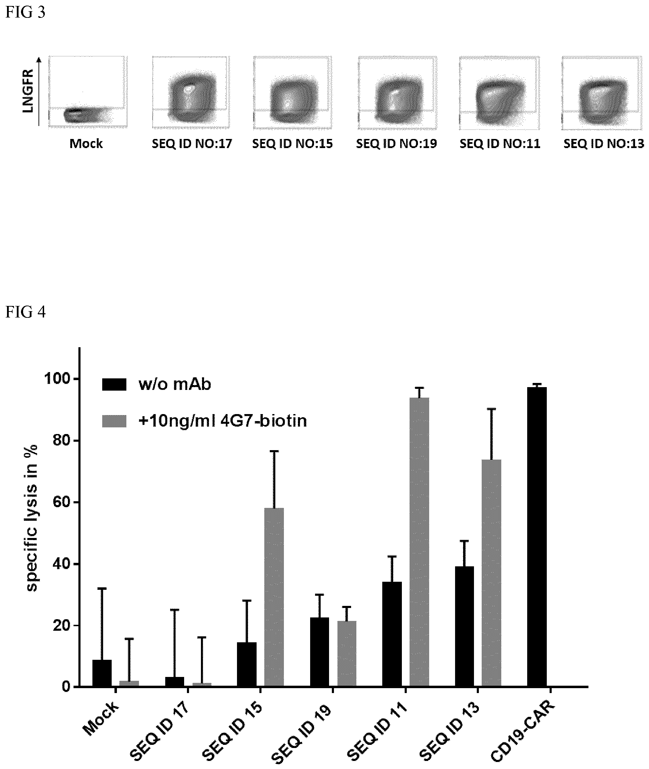

FIG 3: LNGFR transgene expression on primary T cells on day 8 after lentiviral vector gene transfer of the different CAR constructs (SEQ ID NO: 17, 15, 19, 11, and 13).
FIG 4: Luciferase-based cytotoxicity assay of non-transduced (Mock), CD19 CAR transduced and LLE-CAR transduced (SEQ ID NO:17, 15, 19, 11, and 13) T cells in presence or absence of 10 ng/ml anti-CD 19-biotin (4G7-biotin) antibody. Effector to target cell ratio was 1:1 in all cases. Specifc lysis after 24h of co-culture is shown, n=4.

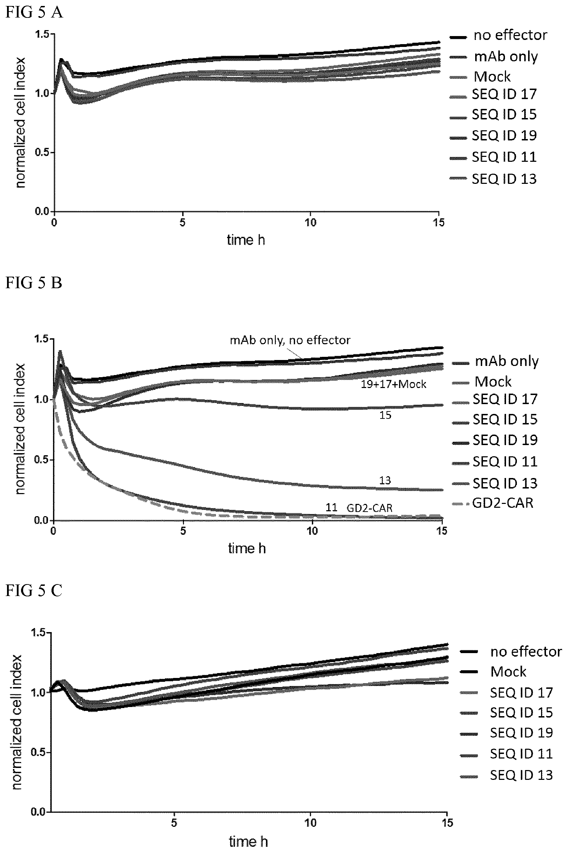

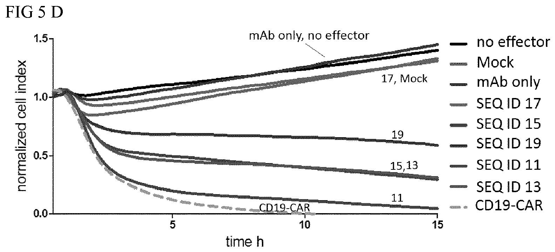

FIG 5: Impedance based real-time cytotoxicity assay (RTCA xCELLigence). Non-transduced (Mock) T cells (A) and anti-LLE CAR transduced T cells (B) (SEQ ID NO:17, 15, 19, 11, and 13) were tested vs. LS cells in presence of lOng/ml anti-GD2-mAB-biotin. Non-transduced (Mock) T cells (C) and anti-LLE CAR transduced T cells (D) (SEQ ID NO: 17, 15, 19, 11, and 13) were tested versus MCF7-tCD19 cells in presence of lOng/ml anti-CD19-biotin antibody. All experiments were done at an effector : target cell ratio of 1:1, with n=3

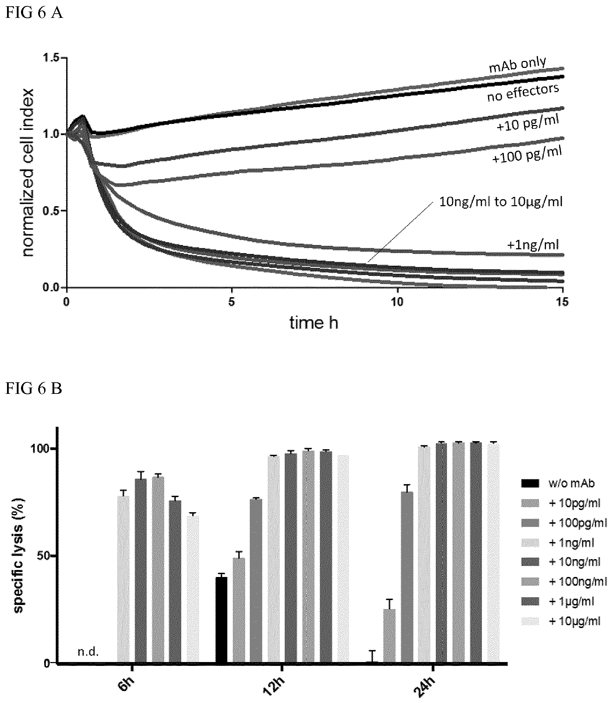

FIG 6: anti-LLE CAR (SEQ ID NO:11) activation and lysis is antibody dose dependent. (A) Impedance-based real-time cytotoxicity assay (RTCA xCELLigence) with anti-LLE CAR versus LS using different anti-GD2-mAB-biotin concentrations at an effector : target cell ratio of 1:1, n=3. (B) Luciferase-based cytotoxicity assay with anti-LLE CAR (SEQ ID NO:11) versus NALM6 with different anti-CD 19-mAB-biotin concentrations.

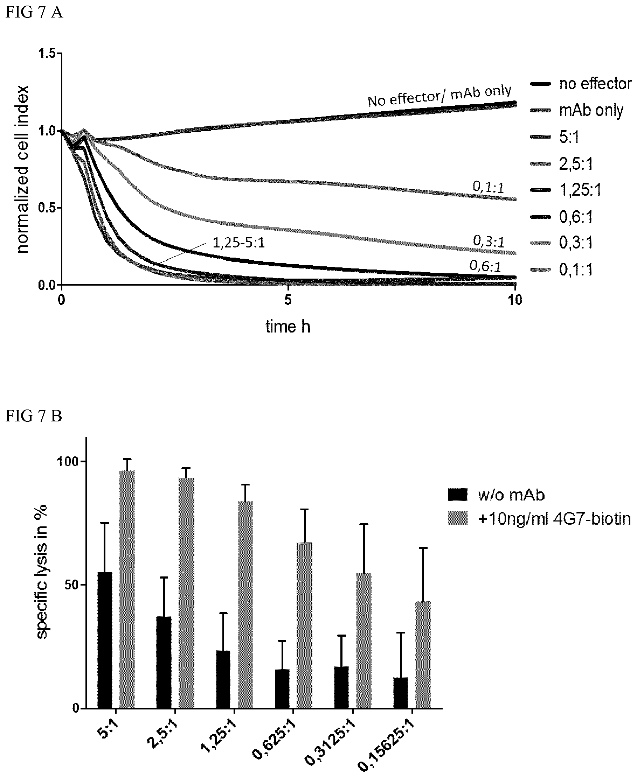

FIG 7: anti-LLE CAR activation and lysis is effector:target cell ratio dependent. (A) Impedance-based real-time cytotoxicity assay (RTCA xCELLigence) with anti-LLE CAR (SEQ ID NO:11) versus LS in presence of 10 ng/ml anti-GD2-mAB-biotin antibody at different effector : target cell ratios, n=3. (B) Luciferase-based cytotoxicity assay (12h) with anti-LLE CAR (SEQ ID NO:11) versus NALM6 at 10 ng/ml anti-CD19-mAB-biotin antibody at different effector : target cell ratios, n=5.

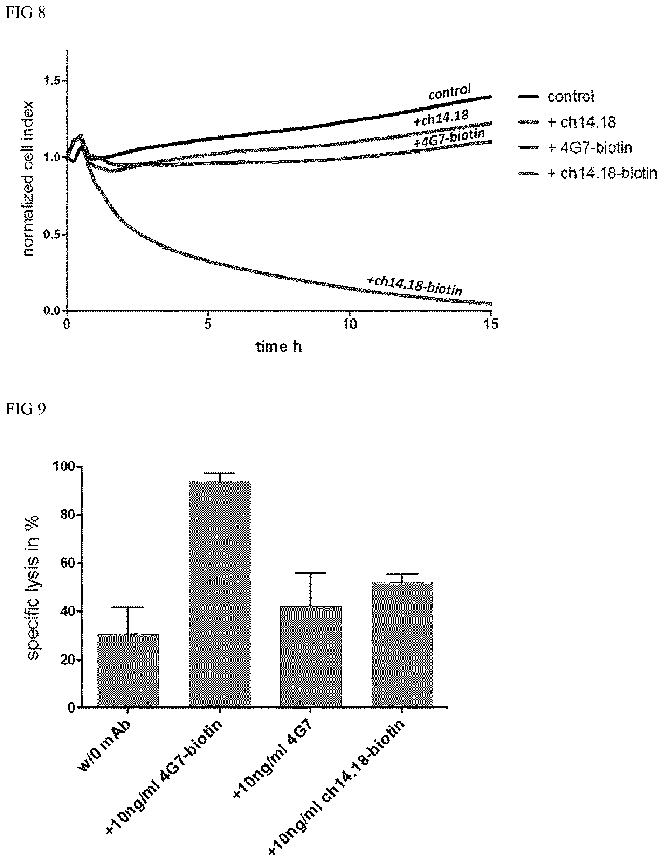

FIG 8: anti-LLE CAR (SEQ ID NO: 11) activation and target cell lysis is antigen and biotin specific: Impedance based real-time cytotoxicity assay (RTCA xCELLigence) with anti-LLE CAR versus LS in presence and absence of lOng/ml anti-GD2-biotin (ch14.18-biotin), anti-GD2 (ch14.18) or target-cell irrelevant anti-CD19-biotin (4G7-biotin) at an effector : target cell ratio of 1:1, n=3.
FIG 9: Specifc lysis of NALM6 target cells by anti-LLE CAR T cells (SEQ ID NO:11) in presence of anti-CD19-biotin (4G7-Biotin), anti-CD19 (4G7), or in presence of target-cell irrelevant anti-GD2-biotin (ch14.18-biotin) at an effector : target cell ratio of 1:1, measured by luciferase-based cytotoxicity assay after 24h, n=4.

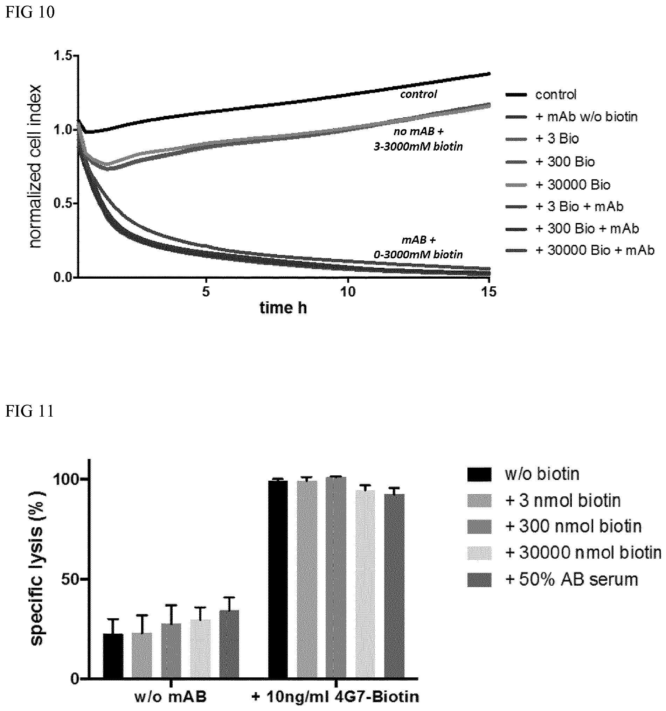

FIG 10: Supraphysiological biotin concentrations do not interfere with anti-LLE CAR activation: Impedance based real-time cytotoxicity assay (RTCA xCELLigence) of anti-LLE CAR T cells (SEQ ID NO:11) versus LS in presence (mAB) or absence (no mAB) of lOng/ml anti-GD2-biotin (ch14.18-biotin) antibody and at different concentrations of free biotin (as indicated).
FIG 11: Specific lysis of NALM6 cells measured after 12 h in presence of anti-LLE CAR T cells and in presence and absence of anti-CD19-biotin (4G7-biotin) antibody and variable concentrations of free biotin or human AB serum.

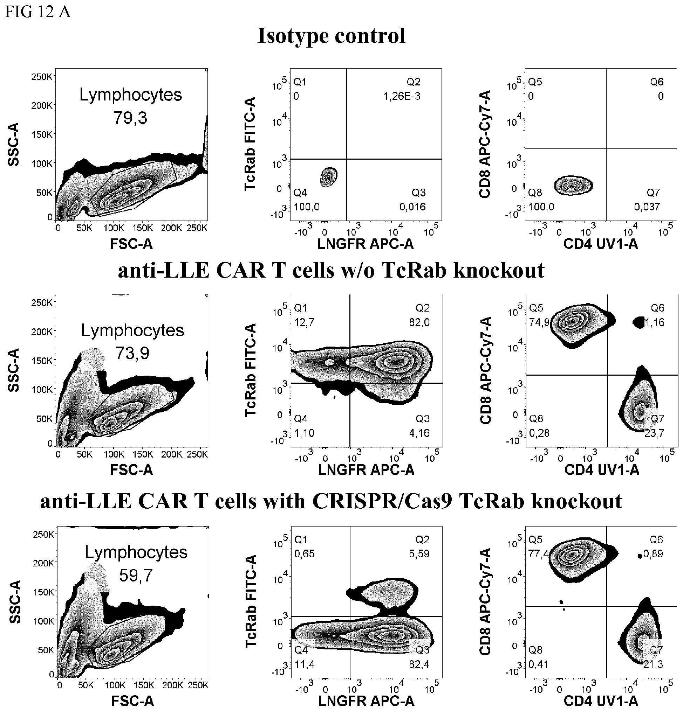

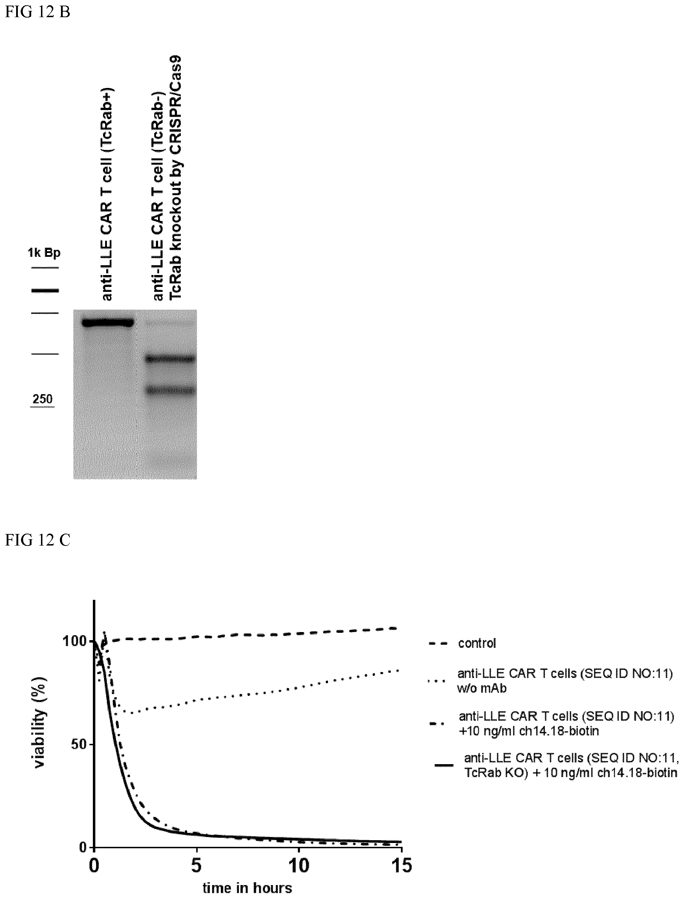

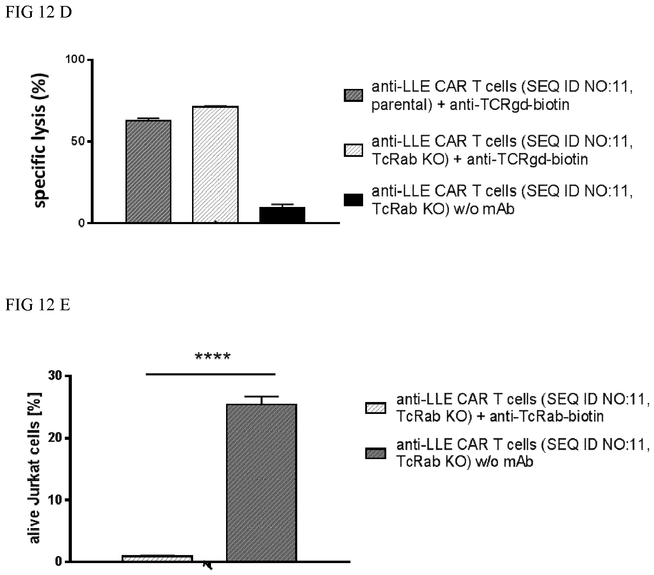

FIG 12: (A) Flow cytometry analysis (anti-TCRa/p-FITC, Miltenyi Biotec) of anti-LLE CAR T cells and anti-LLE CAR T cells knocked out for TcRαβ generated by CRISPR/Cas9 technology. (B) TcRαβ knockout was confirmed by TIDE. (C) Impedance based real-time cytotoxicity assay (RTCA xCELLigence) of anti-LLE CAR T cells (SEQ ID 11) and TcRαβ knockout anti-LLE CAR T cells (SEQ ID NO:11) versus LS in presence (mAB) and absence (mAB) of lOng/ml anti-GD2-biotin (ch14.18-biotin) antibody. (D) Specifc lysis of MOLT14 target cells by anti-LLE CAR T cells (SEQ ID 11) and TcRαβ knockout anti-LLE CAR T cells (SEQ ID NO:11) in presence and absence of 10 ng/ml anti-TcRγδ-biotin mAB, at an effector : target cell ratio of 1:1, measured by luciferase-based cytotoxicity assay after 48h, n=1, 4 technical replicates. (E) Relative survival of JURKAT target cells incubated with anti-LLE CAR T cells (SEQ ID NO:11) and TcRαβ knockout anti-LLE CAR T cells (SEQ ID NO:11) in presence and absence of 10 ng/ml anti-TcRαβ-biotin mAB, at an effector : target cell ratio of 1:1, measured by flow cytometry after 48h, n=1, 4 technical replicates.

### Detailed description of the invention

The present disclosure provides an adaptable CAR system for targeting different and varying antigens on target cells, wherein the CAR binds with a higher preference to a modified self antigen than to the self antigen in a subject minimizing the risk of autoimmunity in a subject in which the CAR system as disclosed herein is used.

In a first aspect the disclosure provides an anti-linker/label epitope chimeric antigen receptor (anti-LLE CAR) comprising
I) an extracellular linker/label epitope (LLE) binding domain,
II) a transmembrane domain, and
III) a signal transduction domain,
   wherein said extracellular LLE binding domain binds a target cell binding molecule (TCBM) comprising
   i) an antigen binding moiety (ABM), wherein said ABM binds specifically to an antigen expressed on a target cell,
   ii) a label moiety (LaM), wherein said LaM is a naturally occurring molecule or a derivative thereof in a subject,
   iii) a linker moiety (LiM) conjugating said ABM and said LaM, thereby forming a linker/label epitope (LLE),
   wherein said extracellular LLE binding domain binds said LLE with a higher preference than said naturally occurring molecule.

Said anti-LLE CAR, wherein said extracellular LLE binding domain binds with an at least twofold higher affinity to said LLE than to the said naturally occurring molecule.

Said anti-LLE CAR, wherein the k(off) value for the binding between said extracellular LLE binding domain is higher to a monomeric LLE and to said naturally occurring molecule than to a multimeric LLE.

Said anti-LLE CAR, wherein said naturally occurring molecule is an extracellular molecule or a molecule with partial extracellular structure.

Said anti-LLE CAR, wherein said naturally occurring molecule is a molecule of the circulatory system of said subject.

Said anti-LLE CAR, wherein said LLE is generated site-specifically, thereby forming an epitope comprising a part of said LaM and a part of said LiM.

Said anti-LLE CAR, wherein said LaM may be a self antigen of said subject.

Said anti-LLE CAR, wherein said LaM may be selected from the group consisting of amino acids, peptides, proteins, creatinine, biotin, biocytin, lipids, hormones, vitamins, carbohydrates, or derivatives thereof. Said lipids may be steroid lipids such as cholesterol.

Said carbohydrates may be e.g. glucose, galactose, or fucose. An especially preferred LaM may be biotin or a derivative thereof.

Said anti-LLE CAR, wherein said LiM is a molecule capable of generating said LLE.

Said anti-LLE CAR, wherein said LiM may be selected from the group consisting of peptides, proteins, nucleic acids, carbohydrates, polyhydroxyalkanoates, alkyl chains, alkanoic acids, carboxylic acids, farnesyls, polyethylene glycols, lipids, or derivatives thereof. Said carboxylic acids acids may be e.g. ε-aminocaproic acid (6-aminohexanoic acid) or 6-(6-aminohexanamido)hexanoic acid).

An especially preferred LiM may be a 6-(6-aminohexanamido) hexanoyl moiety, e.g. derived from 6-(6-aminohexanamido)hexanoic acid or 6-(6-aminohexanamido)hexanoic active ester, or a 6-aminohexanoyl moiety, e.g. derived from 6-aminohexanoic acid or 6-aminohexanoic active ester.

Said anti-LLE CAR, wherein said LaM is biotin and said LiM is a 6-(6-aminohexanamido) hexanoyl linker moiety or a 6-aminohexanoyl linker moiety.

Said anti-LLE CAR, wherein said extracellular LLE binding domain comprises the sequence of SEQ ID NO:1 and SEQ ID NO:2, thereby binding to a 6-[6-(biotinamido)hexanamido] hexanoyl moiety.

Said anti-LLE CAR, wherein said antigen that is bound by said ABM is expressed on a cancer cell.

Said anti-LLE CAR, wherein said antigen may be selected from the group consisting of CD33 (Siglec-3), CD123 (IL3RA), CD135 (FLT-3), CD44 (HCAM), CD44V6, CD47, CD184 (CXCR4), CLEC12A (CLL1), LeY, FRβ, MICA/B, CD305 (LAIR-1), CD366 (TIM-3), CD96 (TACTILE), CD133, CD56, CD29 (ITGB1), CD44 (HCAM), CD47 (IAP), CD66 (CEA), CD112 (Nectin2), CD117 (c-Kit), CD133, CD146 (MCAM), CD155 (PVR), CD171 (L1CAM), CD221 (IGF1), CD227 (MUC1), CD243 (MRD1), CD246 (ALK), CD271 (LNGFR), CD19, CD20, GD2, and EGFR.

Said anti-LLE CAR, wherein said ABM may be an antibody or an antigen-binding fragment thereof.

Said anti-LLE CAR, wherein said anti-LLE CAR comprises the sequence of SEQ ID NO:11, SEQ ID NO:12, SEQ ID NO:13, SEQ ID NO:14, SEQ ID NO:15, or SEQ ID NO:16, thereby binding to a 6-[6-(biotinamido)hexanamido] hexanoyl moiety. Preferentially said anti-LLE CAR comprises the sequence of SEQ ID NO:11, or SEQ ID NO:12.

Said anti-LLE CAR, wherein said anti-LLE CAR is expressed in a eukaryotic cell.

Said anti-LLE CAR, wherein said anti-LLE CAR is expressed in an immune cell.

Said anti-LLE CAR, wherein said anti-LLE CAR is expressed in a T cell, NK cell, NKT cell, gamma/delta T cell or Treg cell.

Said anti-LLE CAR, wherein said anti-LLE CAR is expressed in a stem cell, multi-lineage progenitor cell or induced pluripotent stem cell that will then differentiate into an immune cell, e.g. T cell that will express the anti-LLE CAR.

In another aspect the disclosure provides an isolated polynucleotide or polynucleotides encoding the anti-LLE CAR as disclosed herein.

In a further aspect the disclosure provides a vector comprising the polynucleotide or vectors comprising the polynucleotides encoding the anti-LLE CAR as disclosed herein.

Said vector, wherein said polynucleotide is operatively linked to at least one regulatory element for expression of said anti-LLE-CAR encoded by said polypeptide.

Said vector or said vectors, wherein said vector(s) is/are a viral vector(s).

Said vector or said vectors, wherein said viral vector(s) is/are retroviral vector(s) or lentiviral vector(s).

The nucleic acids molecule(s) may be constructed so that they encode at least all embodiments of the CARs disclosed herein. There may be a contiguous nucleic acid molecule when all domains of the CAR encoded by said molecule are within one polypeptide.

Alternatively, there may be two or more nucleic acid molecules e.g. when some domains of the CAR encoded by said molecules are on separate polypeptides.

In a further aspect the disclosure provides an isolated cell comprising the anti-LLE CAR as disclosed herein.

Said isolated cell may be a eukaryotic cell.

Said isolated cell may be an immune cell such as e.g. a T cell, NK cell, NKT cell, gamma/delta T cell or Treg cell.

Said isolated cell may be a stem cell, multi-lineage progenitor cell or induced pluripotent stem cell that will then differentiate into an immune cell, e.g. T cell that will express the anti-LLE CAR.

Said isolated cell may be a T cell, wherein optionally said T cell does not express TcRαβ, PD1, CD3 or CD96 (e.g. by way of knocking out one of these genes).

Said isolated cell may be an immune cell, wherein optionally said cell does not express accessory molecules that can be checkpoint, exhaustion or apoptosis associated signaling receptors as well as ligands such as PD-1, LAG-3, TIGIT, CTLA-4, FAS-L and FAS-R, (e.g. by way of knocking out one of these genes).

In a further aspect the disclosure provides an isolated cell comprising the polynucleotide(s) encoding the anti-LLE CAR as disclosed herein.

In a further aspect the disclosure provides an isolated cell comprising the vector(s) comprising the polynucleotides encoding the anti-LLE CAR as disclosed herein.

In one aspect the invention provides a composition comprising
a) a target cell binding molecule (TCBM) comprising
   i) an antigen binding moiety (ABM), wherein said ABM binds specifically to an antigen expressed on a target cell,
   ii) a label moiety (LaM), wherein said LaM is a naturally occurring molecule in a subject or a derivative thereof, wherein said naturally occurring molecule is in the circulatory system of said subject,
   iii) a linker moiety (LiM) conjugating said ABM and said LaM, thereby forming a linker/label epitope (LLE), wherein said LiM is selected from the group consisting of peptides, alkyl chains, alkanoic acids, carboxylic acids, farnesyls, polyethylene glycols, or a derivative thereof; and
b) an anti-linker/label epitope (anti-LLE) chimeric antigen receptor (CAR), said anti-LLE CAR comprising
   I) an extracellular linker/label epitope (LLE) binding domain,
   II) a transmembrane domain, and
   III) a signal transduction domain,
   wherein said extracellular LLE binding domain binds said LLE with a higher preference than said naturally occurring molecule, wherein said extracellular LLE binding domain binds with an at least 10-fold higher affinity to said LLE than to said naturally occurring molecule.

All characteristics of the TCBM and the anti-LLE CAR described above may also apply in said composition.

In another aspect the disclosure provides a method of treating a disorder in a subject, comprising
a) administering one or more formulations of a target cell binding molecule (TCBM) to a subject in need of treatment, said TCBM comprising
   i) an antigen binding moiety (ABM), wherein said ABM binds specifically to an antigen expressed on a target cell,
   ii) a label moiety (LaM), wherein said LaM is a naturally occurring molecule in a subject or a derivative thereof,
   iii) a linker moiety (LiM) conjugating said ABM and said LaM, thereby forming a linker/label moiety epitope (LLE), and
b) administering to the subject one or more therapeutically effective populations of anti-LLE CAR expressing cells, said anti-LLE CAR comprising
   I) an extracellular linker/label epitope (LLE) binding domain,
   II) a transmembrane domain, and
   III) a signal transduction domain,
   wherein said extracellular LLE binding domain binds said LLE with a higher preference than said naturally occurring molecule, thereby inducing cell death of said target cell.

All characteristics of the TCBM and the anti-LLE CAR described above may also apply in said method for treating a disorder in a subject.

Said method, wherein said treatment of a disorder is a cancer, and wherein said target cell is a cancer cell.

Said method, wherein said more formulations of TCBM are the same or different formulations of TCBM.

Said method, wherein said more therapeutically effective populations of anti-LLE CAR expressing cells are the same or different populations of anti-LLE CAR expressing cells.

Said method, wherein said anti-LLE CAR of said more therapeutically effective populations are the same or different anti-LLE CARs.

Said method, wherein said administering of said more formulations of a target cell binding molecule (TCBM) to a subject in need of treatment are administered simultaneously or subsequently.

Said method, wherein said administering of said more therapeutically effective populations of an anti-LLE CAR expressing cells are administered simultaneously or subsequently.

The present disclosure is directed to a CAR system for use in the treatment of subjects suffering from a disorder such as cancer. The CAR system of the present invention (e.g., immune cells such as T cells expressing the CARs comprising an LLE binding domain and corresponding target cell binding molecules (TCBM)) makes use of CARs that target an epitope that does not occur in the subject being treated, i.e. the modified self antigen. As the label moiety used in this system is a naturally occurring molecule in the subject, i.e. a self antigen, it is non-immunogenic. The epitope that does not naturally occur in the subject is generated by the coupling of the label moiety with a linker moiety creating a new interface and/or environment at the linkage of label moiety and linker moiety so that a new epitope recognizing molecule may be generated that may be used as a linker/label epitope binding domain of a CAR of the system as disclosed herein.

This CAR system as disclosed herein thus allows for focused targeting of the cells such as immune cells to target cells expressing an antigen, such as cancer cells.

The linker moiety and label moiety are part of the target cell binding molecule (TCBM) that also comprises an antigen binding moiety (ABM), wherein the linker moiety conjugates the LaM and the ABM. Generally, said ABM is directed against an antigen expressed on the surface of a target cell.

By administration of TCBM along with the CAR-expressing immune cells, the immune cell response can be targeted to only those cells expressing the antigen on the surface of the target cells, thereby reducing off-target toxicity. The CAR-expressing immune cells can be used as "universal" immune cells to target a wide variety of target cells, e.g. a wide variety of tumors without the need to prepare separate CAR constructs. The label/linker epitope of the TCBM recognized by the CAR may also remain constant. It is only the ABM of the TCBM that needs to be altered to allow the system to target target cells of different identity.

The following illustrates exemplary the CAR system disclosed herein:
Biotin is a naturally occurring molecule in the circulatory system of a subject, e.g. a human.

Therefore, biotin is non-immunogenic for said subject.

A TCBM is prepared that comprises biotin as a label moiety coupled (conjugated) to the 6-(6-aminohexanamido) hexanoyl linker moiety resulting in a 6-[6-(biotinamido)hexanamido] hexanoyl moiety.

The TCBM comprises also an ABM, e.g. an antibody or scFv specific for an antigen on the surface of a target cell, such as GD-2 or CD19 that are antigens expressed on cancer cells, wherein the 6-(6-aminohexanamido) hexanoyl linker moiety bridges the biotin and the ABM. Immune cells are transduced to express a CAR that comprises a 6-[6-(biotinamido)hexanamido] hexanoyl moiety binding domain as an LLE binding domain. This CAR thus targets above-mentioned TCBM.

The two components, i.e. the TCBM and the immune cells expressing the CAR, are administered to a subject suffering from a disorder such as cancer. The TCBM is bound by the target cells, e.g. the cancer cells (through binding of the ABM portion of the TCBM to cognate target cell antigens such as GD-2 or CD19). The 6-[6-(biotinamido)hexanamido] hexanoyl moiety of the TCBM is then recognized and bound by the anti-6-[6-(biotinamido)hexanamido] hexanoyl moiety CAR expressed by the immune cells. Upon 6-[6-(biotinamido)hexanamido] hexanoyl-ABM conjugate binding, the anti-6-[6-(biotinamido)hexanamido] hexanoyl moiety CAR-expressing immune cells are activated and the target cell, e.g. the cancer cell may be killed. The anti-6-[6-(biotinamido)hexanamido] hexanoyl moiety CAR binds with higher preference 6-[6-(biotinamido)hexanamido] hexanoyl cojugates than to the circulating biotin in the subject.

The CAR-expressing immune cells as disclosed herein cannot kill cells by direct binding to a target cell such as a cancer cell. The TCBM thus acts as a bridge between the CAR-expressing immune cells and the target cells. As long as the label/linker moiety that generates the linker/label epitope of the TCBM is a moiety not found naturally in the subject and the extracellular LLE binding domain of the CAR binds said LLE with a higher preference than said naturally occurring molecule, the activity of the CAR-expressing immune cells can be limited to the target cells. Further, the activation of the CAR-expressing immune cells can be regulated by limiting the amount of TCBM administered to a subject, for example, by manipulating infusion of the TCBM if a side effect is detected.

The CAR system of the present disclosure utilizes TCBMs as the bridge between immune cells expressing the CAR and target cells expressing the antigen. The TCBM comprises a label moiety (LaM) on one end of the molecule and an antigen binding moiety (ABM) on the other end, connected by a linker moiety. The sole requirement for the identity of the label moiety is only in that it must be a naturally occurring molecule in a subject (a self antigen) and that the linker moiety conjugated variant thereof can be recognized and bound by a CAR expressed by an immune cell with higher affinity to the linker moiety conjugated variant thereof (the modified self antigen) than to the non-linker moiety conjugated, naturally occurring variant.

The ABM may comprise single antigen specificity (monospecific), two or more antigen specificities (bi- and multispecific). An example for a bispecific ABM is an ABM that binds to the antigens CD19 and CD20.

The ABM may also comprise monovalent binding as well a bi- and multivalent binding site. An example for a bivalent ABM is a ABM that comprises two scFvs recognizing different epitopes of an antigen.

The ABM can be an immunoglobulin against the target derived from immunization of a host (e.g. mouse). Furthermore, it can also be derived from rational design and recombinant expression. In another embodiment the ABM can also be derived from methods such as directed evolution and screening techniques such as phage display and ribosome display.

The label moiety and the antigen binding moiety may be covalently conjugated via a linker moiety, e.g the chemical coupling of biotin and 6-(6-aminohexanamido) hexanoyl moiety.

Alternatively, the label moiety and the antigen binding moiety may be non-covalently conjugated via a linker moiety. In addition, alternatively, the TCBM may comprise further moieties or domains between ABM and LiM. The sole requirement is that LiM is conjugated (or coupled) to LaM in such a way that it allows the generation of the linker/label epitope as disclosed herein.

The linker moiety may be conjugated to the label moiety resulting in a moiety comprising the LLE. The LiM, coupled to the LaM can be coupled randomly e.g. by targeting lysines on a protein ABM using an e.g. amino reactive succinimidyl ester derivative of the LiM.

Alternatively, the linker moiety may be conjugated to the ABM site-specifically, e.g. by incorporating an unnatural aminoacid in a recombinatly expressed protein ABM using e.g. a nonsense suppressor tRNA delivering said unnatural aminoacid at an engineered stop codon in the coding sequence of said ABM.

Every molecule that might be capable of generating a LLE as disclosed herein may be used as a linker moiety. The sole requirement for the identity of a linker moiety is that the linker moiety may be chemically conjugated (or coupled) to a label moiety or genetically (recombinatly) encoded and should be able to generate a new epitope at the context, the interface and/or environment of linker moiety and label moiety as disclosed herein. The LiM may be preferentially a molecule that does not evoke or does not tend to evoke an immune reaction in the subject, e.g. the LiM is a self antigen. In this case the interface of the LaM and LiM generates a novel epitope, the LLE.

The LiM may be e.g selected from the group consisting of peptides, proteins, nucleic acids, carbohydrates, polyhydroxyalkanoates, alkyl chains, alkanoic acids, carboxylic acids (e.g. ε-aminocaproic acid (6-aminohexanoic acid) or 6-(6-aminohexanamido)hexanoic acid) farnesyls, polyethylene glycols, lipids and derivatives thereof.

An especially preferred LiM may be a 6-(6-aminohexanamido) hexanoyl moiety, e.g. derived from 6-(6-aminohexanamido)hexanoic acid or 6-(6-aminohexanamido)hexanoic active ester, or a 6-aminohexanoyl moiety, e.g. derived from 6-aminohexanoic acid or 6-aminohexanoic active ester.

Said anti-LLE CAR, wherein said LaM is biotin and said LiM is a 6-(6-aminohexanamido) hexanoyl linker moiety or a 6-aminohexanoyl linker moiety.

The skilled artisan will understand and recognize that various means can be used to prepare TCBMs comprised of a label moiety, a linking moiety, and an antigen binding moiety.

Prior to being administered to a subject, the TCBMs may be prepared in a pharmaceutically acceptable formulation. Such formulations may contain a pharmaceutically acceptable excipient or carrier.

The affinity at which the ABM binds the antigen on the target cell may vary, but generally the binding affinity may be at least about 100 µM, 1 nM, 10 nM, or 100 nM, preferably at least about 1 pM or 10 pM, even more preferably at least about 100 pM.

It is a requirement that the extracellular LLE binding domain of the CAR as disclosed herein binds the LLE of the TCBM with a higher preference than the corresponding naturally occurring molecule.

Said extracellular LLE binding domain of the CAR may be produced using commercially available antibodies or antigen binding fragments thereof.

Alternatively, antibodies or antigen recognizing molecules for a selected LLE may be newly produced by methods well-known to the skilled person.

The affinity at which the LLE binding domain of the CAR binds to the LLE of the TCBM can vary, but generally the binding affinity may be in the range of 0,01nM to 1µM, preferentially in the range of 0,1 -100 nM, or more preferentially in the range of 1-30 nM. The sole requirement is that the affinity at which the LLE binding domain of the CAR binds to the LLE of the TCBM has to be higher than the affinity at which the LLE binding domain of the CAR bind the naturally occurring molecule (i.e. without linker moiety) in the subject. Preferentially, the extracellular LLE binding domain binds with an at least twofold, preferentially at least 5-fold, more preferentially at least 10-fold, most preferentially at least 20-fold higher affinity to said LLE than to the said naturally occurring molecule.

The DNA or RNA construct(s) (nucleic acid molecule(s)) encoding the CAR of the invention can be transfected or transduced into a host cell by methods well known in the art (e.g. viral-based systems including retrovirus and lentivirus, physical methods including electroporation, biological methods, chemical methods). Regardless the methods used to integrate, preferentially stably integrate, the DNA encoding the CAR of the invention, in the host cell, as a result the host cell expresses a CAR as disclosed herein.

Alternatively, the nucleic acid sequences may be produced synthetically.

An engineered cell expressing a CAR as disclosed herein may be isolated (enriched or separated) after the transfection/transduction process for generating such an engineered CAR cell from non-transfected/transduced cells by methods well known in the art, e.g. fluorescent based separating technologies such as FACS® or magnetic cell separation methods such as MACS® (Miltenyi Biotec GmbH).

Generally, the cells such as immune cells, preferentially T cells for generating engineered cells expressing the CAR of the invention may be obtained from a subject. Cells such as immune cells, preferentially T cells, can be obtained from a variety of sources such as peripheral blood mononuclear cells (PMBCs), bone marrow, lymph node tissue, cord blood or thymus tissue. For enrichment of these cells methods well known in the art can be used such as centrifugation through a Ficoll™ or PERCOLL™ gradient or positive/negative selection techniques such as fluorescent sorting (e.g. FCASsort) or magnetic sorting (e.g. MACS®). Preferably the enriched cell population comprises at least about 90% of the selected cell type. In particular aspects, the cell population comprises at least about 90%, 91%, 92%, 93%, 94%, 95%, 96%, 97%, 98%, 99%, or even 100% of the selected cell type.

Exemplary, T cells of a blood sample of a subject are magnetically labelled, for example with a magnetic bead coupled to antibodies specific for CD4, washed, magnetically enriched and collected. Then these T cells may be engineered to express the CAR as disclosed herein on their cell surface.

In one embodiment of the disclosure the isolated/enriched engineered cell such as immune cells, preferentially T cells expressing the CAR as disclosed herein may be activated prior or after genetic modification and expanded to increase the number of engineered (T) cells using methods well known in the art, for example polyclonal stimulation of T cells with the TransAct T Cell Reagent (Miltenyi Biotec; WO2014048920A1) that consists of a colloidal nanomatrix conjugated to humanized CD3 and CD28 agonists. Preferentially, said number of engineered cells such as immune cells, e.g. T cells, may be increased to a therapeutically effective amount.

A cell expressing the CAR as disclosed herein may be engineered by a RNA encoding the CAR of the invention. The RNA may be transfected or transduced into a host cell by methods well known in the art (e.g. viral-based systems, physical methods, biological methods, chemical methods). Regardless the methods used to transfer the RNA encoding the CAR of the invention into the host cell, as a result the host cell expresses a CAR as disclosed herein. "RNA-engineered cells" express the CAR for a limited time (transient expression).

The genetically modified (immune) cells expressing the CAR as disclosed herein, preferentially T cells, may be generated in an automated process in a closed system. A process for the generation of genetically modified cells, preferentially T cells, may comprise e.g. the steps:
a) providing a cell sample comprising (immune) cells
b) preparation of the cell sample by centrifugation
c) magnetic separation of the (immune) cells, preferentially T cells,
d) activation of the enriched (immune) cells, preferentially T cells, using modulatory agents
e) genetically modifying the (immune) cells, preferentially T cells, to express the CAR of the invention
f) expansion of the genetically modified (immune) cells, preferentially T cells, in a cultivation chamber
g) washing of the cultured (immune) cells, preferentially T cells.

All these steps may be performed automatically in a closed system, preferentially in a closed and sterile system.

The process is especially suited for preparing gene modified cells such as immune cells, preferentially T cells, wherein the enriched (immune) cells, preferentially T cells, are gene-modified by using viral and/or non-viral vectors.

Any of these steps may be multiplied, omitted or may occur in a different order.

The modulatory agents may be selected from agonistic antibodies and / or cytokines.

The gene-modified (immune) cells, preferentially T cells, may be enriched by magnetic labelling of (immune) cells and magnetic separation before or after cultivation to obtain higher frequency of gene-modified (immune) cells, preferentially T cells, in the final cellular product.

As a closed and sterile system for cell modification, the fully automated cell processing device CliniMACS Prodigy® and associated tubing sets (Miltenyi Biotec GmbH, Germany) may be used (WO2009/072003). This closed system meets the requirements of GMP-grade processing of almost any kind of cellular products and may allow reducing clean room requirements, improve technology transfer and harmonization of cell manufacturing processes.

The CAR system as disclosed herein may be used for the treatment in a subject having a disease, a disorder or a condition associated with an antigen which can be bound specifically by the antigen binding moiety of the TCBM as disclosed herein.

In particular, the CAR system as disclosed herein may be for use in the treatment in a subject suffering from cancer. Cells such as immune cells, e.g. T cells of a subject, may be isolated or established immune cell lines may be used. The subject may suffer from said cancer (a patient) or may be a healthy subject. These immune cells are genetically modified *in vitro* to express the CAR as disclosed herein. These engineered cells may be activated and expanded *in vitro* to a therapeutically effective population of anti-LLE CAR expressing cells. In cellular therapy these engineered cells may be infused to a recipient in need thereof as a pharmaceutical composition (or a formulation of a therapeutically effective population of anti-LLE CAR expressing cells), in addition to a second pharmaceutical composition, a formulation of TCBM. The infused cells in the recipient may be able to kill (or at least stop growth of) cancerous cells expressing the antigen which is recognized by the CAR system as disclosed herein. The recipient may be the same subject from which the cells were obtained (autologous cell therapy) or may be from another subject of the same species (allogeneic cell therapy).

The therapeutically effective population of anti-LLE CAR expressing cells may be administered to the patient before the administration of the formulation of TCBM to the subject. Alternatively, the formulation of TCBM may be administered to the subject before or at the same time as the administration the therapeutically effective population of anti-LLE CAR expressing cells to the subject. A further variation includes *in-vitro* culturing the therapeutically effective population of anti-LLE CAR expressing cells with the TBCM of the formulation of TCBM prior to administration to the subject.

Populations of anti-LLE-CAR-expressing (immune) cells may be formulated for administered to a subject using techniques known to the skilled artisan.

Formulations comprising therapeutically effective population(s) of anti-LLE expressing CAR cells may include pharmaceutically acceptable excipient(s) (carrier or diluents). Excipients included in the formulations will have different purposes depending, for example, on the nature of the LLE-binding domain of the anti-LLE-CAR, the (sub)population of immune cells used, and the mode of administration. Examples of generally used excipients include, without limitation: saline, buffered saline, dextrose, water-for-injection, glycerol, ethanol, and combinations thereof, stabilizing agents, solubilizing agents and surfactants, buffers and preservatives, tonicity agents, bulking agents, and lubricating agents.

A formulation of a therapeutically effective population(s) of anti-LLE expressing CAR cells may include one population of anti-LLE-CAR-expressing (immune) cells, or more than one population of anti-LLE-CAR-expressing (immune) cells. The different populations of anti-LLE-CAR (immune) cells may vary based on the identity of the LLE-binding domain, the identity of the activation domain, the identity of the (sub)population of immune cells, or a combination thereof.

The formulations comprising therapeutically effective population(s) of anti-LLE expressing CAR cells may be administered to a subject using modes and techniques known to the skilled artisan. Exemplary modes include, but are not limited to, intravenous injection. Other modes include, without limitation, intratumoral, intradermal, subcutaneous (s.c, s.q., sub-Q, Hypo), intramuscular (i.m.), intraperitoneal (i.p.), intra-arterial, intramedulary, intracardiac, intra-articular (joint), intrasynovial (joint fluid area), intracranial, intraspinal, and intrathecal (spinal fluids).

The formulations comprising therapeutically effective population(s) of anti-LLE expressing CAR cells that are administered to a subject comprise a number of anti-LLE-CAR-expressing cells such immune cells that is effective for the treatment of the specific indication or disorder. In general, formulations may be administered that comprise between about 1 x 10⁴ and about 1 x 10¹⁰ anti-LLE-CAR-expressing cells such as immune cells. In most cases, the formulation may comprise between about 1 x 10⁵ and about 1 x 10⁹ anti-LLE-CAR-expressing cells such as immune cells, from about 5 x 10⁵ to about 5 x 10⁸ anti-LLE-CAR-expressing cells such as immune cells, or from about 1 x 10⁶ to about 1 x 10⁷ anti-LLE-CAR -expressing cells such as immune cells. However, the number of anti-LLE-CAR-expressing cells such as immune cells administered to a subject may vary between wide limits, depending upon the location, source, identity, extent and severity of the disorder, the age and condition of the individual to be treated, etc. A physician may ultimately determine appropriate dosages to be used.

The TCBMs may be formulated for administered to a subject using techniques known to the skilled artisan. Formulations of the TCBMs may include pharmaceutically acceptable excipient(s) (carriers or diluents). Excipients included in the formulations will have different purposes depending, for example, on the nature of the label moiety, the linker moiety, the ABM, and the mode of administration. Examples of generally used excipients include, without limitation: saline, buffered saline, dextrose, water-for-injection, glycerol, ethanol, and combinations thereof, stabilizing agents, solubilizing agents and surfactants, buffers and preservatives, tonicity agents, bulking agents, and lubricating agents.

A formulation of TCBM may include one type of TCBM, or more than one type of TCBM. The different types of TBCMs may vary based on the identity of the label moiety, the linker moiety, the antigen binding moiety, or a combination thereof.

The TCBMs may be administered to a subject using modes and techniques known to the skilled artisan. Exemplary modes include, but are not limited to, intravenous, intraperitoneal, and intratumoral injection. Other modes include, without limitation, intradermal, subcutaneous (s.c, s.q., sub-Q, Hypo), intramuscular (i.m.), intra-arterial, intramedulary, intracardiac, intra-articular (joint), intrasynovial (joint fluid area), intracranial, intraspinal, and intrathecal (spinal fluids).

Formulations comprising the TCBM are administered to a subject in an amount which is effective for treating the specific indication or disorder. In general, formulations comprising at least about 1 µg/kg to about 100 mg/kg body weight of the TCBM may be administered to a subject in need of treatment. In most cases, the dosage may be from about 100 µg/kg to about 10 mg/kg body weight of the TCBM daily, taking into account the routes of administration, symptoms, etc. As shown in FIG 6 surpringly it is possible to use TCBMs in concentrations as low as 1 ng/ml (corresponding to 1 µg/kg body weight) using the CAR system as disclosed herein. However, the amount of TCBM in formulations administered to a subject may vary between wide limits, depending upon the location, source, identity, extent and severity of the disorder, the age and condition of the individual to be treated, etc. A physician may ultimately determine appropriate dosages to be used.

The timing between the administration of the CAR expressing cell formulation and the TCBM -formulation may range widely depending on factors that include the type of (immune) cells being used, the binding specificity of the CAR, the identity of the label moiety, linker moiety and antigen binding moiety, the identity of the target cell, e.g. cancer cell to be treated, the location of the target cell in the subject, the means used to administer the formulations to the subject, and the health, age and weight of the subject being treated. Indeed, the TCBM formulation may be administered prior to, simultaneous with, or after the genetically engineered (immune) cell formulation.

Depending on the disorder being treatment the step of administering the CAR expressing cell formulation, or the step of administering the TCBM formulation, or both, can be repeated one or more times. When two or more formulations of TCBM may be applied to a subject, the formulations applied may comprise the same or different TCBMs. When two or more formulations of engineered cells such as immune cells expressing the CAR of the disclosure are applied to a subject, the engineered cells may be of the same cell type or of different cell types. A formulation of cells such as immune cells may also comprise more than one cell type, each expressing the CAR of the disclosure.

### Definitions

Unless defined otherwise, technical and scientific terms used herein have the same meaning as commonly understood by one of ordinary skill in the art to which this invention belongs.

In general, a CAR may comprise an extracellular domain (extracellular part) comprising the antigen binding domain, a transmembrane domain and an intracellular signaling domain. The extracellular domain may be linked to the transmembrane domain by a linker. The extracellular domain may also comprise a signal peptide. The extracellular part of the CAR of the present invention comprises a linker/label epitope (LLE) binding domain as disclosed herein. Specifically, the CAR as disclosed herein has an extracellular LLE binding domain as antigen binding domain.

A "signal peptide" refers to a peptide sequence that directs the transport and localization of the protein within a cell, e.g. to a certain cell organelle (such as the endoplasmic reticulum) and/or the cell surface.

Generally, an "antigen binding domain" refers to the region of the CAR that specifically binds to an antigen (and thereby is able to target a cell containing the antigen). The CARs of the disclosure may comprise one or more antigen binding domains. Generally, the targeting regions on the CAR are extracellular. The antigen binding domain may comprise an antibody or an antigen binding fragment thereof. The antigen binding domain may comprise, for example, full length heavy chain, Fab fragments, single chain Fv (scFv) fragments, divalent single chain antibodies or diabodies. Any molecule that binds specifically to a given antigen such as affibodies or ligand binding domains from naturally occurring receptors may be used as an antigen binding domain. Often the antigen binding domain is a scFv. Normally, in a scFv the variable regions of an immunoglobulin heavy chain and light chain are fused by a flexible linker to form a scFv. Such a linker may be for example the "(G₄/S₁)₃-linker".

In some instances, it is beneficial for the antigen binding domain to be derived from the same species in which the CAR will be used in. For example, when it is planned to use it therapeutically in humans, it may be beneficial for the antigen binding domain of the CAR to comprise a human or humanized antibody or antigen binding fragment thereof. Human or humanized antibodies or antigen binding fragments thereof can be made by a variety of methods well known in the art. The CAR as disclosed herein has an extracellular linker/label epitope binding domain as an antigen binding domain allowing to bind indirectly via a target cell binding molecule as disclosed herein to an antigen expressed on a target cell.

"Spacer" or "hinge" as used herein refers to the hydrophilic region which is between the antigen binding domain and the transmembrane domain. The CARs of the disclosure may comprise an extracellular spacer domain but is it also possible to leave out such a spacer. The spacer may include e.g. Fc fragments of antibodies or fragments thereof, hinge regions of antibodies or fragments thereof, CH2 or CH3 regions of antibodies, accessory proteins, artificial spacer sequences or combinations thereof. A prominent example of a spacer is the CD8alpha hinge.

The transmembrane domain of the CAR may be derived from any desired natural or synthetic source for such domain. When the source is natural the domain may be derived from any membrane-bound or transmembrane protein. The transmembrane domain may be derived for example from CD8alpha or CD28. When the key signaling and antigen recognition modules (domains) are on two (or even more) polypeptides then the CAR may have two (or more) transmembrane domains. The splitting key signaling and antigen recognition modules enable for a small molecule-dependent, titratable and reversible control over CAR cell expression (Wu et al, 2015, Science 350: 293-303) due to small molecule-dependent heterodimerizing domains in each polypeptide of the CAR.

The cytoplasmic domain (or the intracellular signaling domain) of the CAR is responsible for activation of at least one of the normal effector functions of the immune cell in which the CAR is expressed. "Effector function" means a specialized function of a cell, e.g. in a T cell an effector function may be cytolytic activity or helper activity including the secretion of cytokines. The intracellular signaling domain refers to the part of a protein which transduces the effector function signal and directs the cell expressing the CAR to perform a specialized function. The intracellular signaling domain may include any complete, mutated or truncated part of the intracellular signaling domain of a given protein sufficient to transduce a signal which initiates or blocks immune cell effector functions.

Prominent examples of intracellular signaling domains for use in the CARs include the cytoplasmic signaling sequences of the T cell receptor (TCR) and co-receptors that initiate signal transduction following antigen receptor engagement.

Generally, T cell activation can be mediated by two distinct classes of cytoplasmic signaling sequences, firstly those that initiate antigen-dependent primary activation through the TCR (primary cytoplasmic signaling sequences) and secondly those that act in an antigen-independent manner to provide a secondary or co- stimulatory signal (secondary cytoplasmic signaling sequences, co-stimulatory signaling domain). Therefore, an intracellular signaling domain of a CAR may comprise one or more primary cytoplasmic signaling domain and/or one or more secondary cytoplasmic signaling domain.

Primary cytoplasmic signaling sequences that act in a stimulatory manner may contain ITAMs (immunoreceptor tyrosine-based activation motifs signaling motifs).

Examples of ITAM containing primary cytoplasmic signaling sequences often used in CARs are that are those derived from TCR zeta (CD3 zeta), FcR gamma, FcR beta, CD3 gamma, CD3 delta, CD3 epsilon, CD5, CD22, CD79a, CD79b, and CD66d. Most prominent is sequence derived from CD3 zeta.

The cytoplasmic domain of the CAR may be designed to comprise the CD3-zeta signaling domain by itself or combined with any other desired cytoplasmic domain(s). The cytoplasmic domain of the CAR can comprise a CD3 zeta chain portion and a co-stimulatory signaling region. The co-stimulatory signaling region refers to a part of the CAR comprising the intracellular domain of a co-stimulatory molecule. A co-stimulatory molecule is a cell surface molecule other than an antigen receptor or their ligands that is required for an efficient response of lymphocytes to an antigen. Examples for a co-stimulatory molecule are CD27, CD28, 4-1BB (CD137), OX40, CD30, CD40, PD-1, ICOS, lymphocyte function-associated antigen- 1 (LFA-1), CD2, CD7, LIGHT, NKG2C, B7-H3.

The cytoplasmic signaling sequences within the cytoplasmic signaling part of the CAR may be linked to each other with or without a linker in a random or specified order. A short oligo- or polypeptide linker, which is preferably between 2 and 10 amino acids in length, may form the linkage. A prominent linker is the glycine-serine doublet.

As an example, the cytoplasmic domain may comprise the signaling domain of CD3-zeta and the signaling domain of CD28. In another example the cytoplasmic domain may comprise the signaling domain of CD3-zeta and the signaling domain of CD27. In a further example, the cytoplasmic domain may comprise the signaling domain of CD3-zeta, the signaling domain of CD28, and the signaling domain of CD27.

As aforementioned either the extracellular part or the transmembrane domain or the cytoplasmic domain of a CAR may also comprise a heterodimerizing domain for the aim of splitting key signaling and antigen recognition modules of the CAR.

The CAR as discloded herein, i.e. the CAR comprising a LLE binding domain may be designed to comprise any portion or part of the above-mentioned domains as described herein in any order and/or combination resulting in a functional CAR.

The term "antibody" as used herein is used in the broadest sense to cover the various forms of antibody structures including but not being limited to monoclonal and polyclonal antibodies (including full length antibodies), multispecific antibodies (e.g. bispecific antibodies), antibody fragments, i.e. antigen binding fragments of an antibody, immunoadhesins and antibody-immunoadhesin chimeras, that specifically recognize (i.e. bind) a target antigen. "Antibody fragments" comprise a portion of a full length antibody, preferably the variable domain thereof, or at least the antigen binding site thereof ("an antigen binding fragment of an antibody"). Examples of antibody fragments include Fab (fragment antigen binding), scFv (single chain fragment variable), single domain antibodies, diabodies, dsFv, Fab', diabodies, single-chain antibody molecules, and multispecific antibodies formed from antibody fragments.

As used herein, the term "antigen" is intended to include substances that bind to or evoke the production of one or more antibodies and may comprise, but is not limited to, proteins, peptides, polypeptides, oligopeptides, lipids, carbohydrates, and combinations thereof, for example a glycosylated protein or a glycolipid. The term "antigen" as used herein refers to a molecular entity that may be expressed on a target cell and that can be recognized by means of the adaptive immune system including but not restricted to antibodies or TCRs, or engineered molecules including but not restricted to transgenic TCRs, CARs, scFvs or multimers thereof, Fab-fragments or multimers thereof, antibodies or multimers thereof, single chain antibodies or multimers thereof, or any other molecule that can execute binding to a structure with high affinity.

The term "anti-6-[6-(biotinamido)hexanamido] hexanoyl moiety CAR" as used herein refers to a CAR that binds to a TCBM that has biotin as a LaM, has a 6-(6-aminohexanamido) hexanoyl linker moiety as a LiM derived e.g. from 6-(6-aminohexanamido) hexanoic acid or 6-(6-aminohexanamido) hexanoic active ester, and has an ABM against an antigen of a target cell.

The interface, lingage and/or interaction part of the 6-(6-aminohexanamido) hexanoyl linker moiety and the biotin label moiety creates the linker/label epitope (LLE), the 6-[6-(biotinamido)hexanamido] hexanoyl moiety.

For example, coupling Biotin-LC-LC-NHS (ThermoFisher Scientific, Mw 567,70 g/mol, Cat. No. 21343, CAS-No. 89889-52-1) to an ABM as disclosed herein results in a TCBM as disclosed herein that can be bound by said anti-6-[6-(biotinamido)hexanamido] hexanoyl moiety CAR as disclosed herein.

The 6-(6-aminohexanamido) hexanoyl linker moiety as shown e.g. in FIG 1C may have any functional chemical group such as a carboxyl group, an active ester, an acetamide or maleimide capable for coupling to an ABM as disclosed herein, for example an antibody or fragment thereof using NH2 or SH groups for coupling thereto.

The term "target cells" as used herein refers to cells which express the antigen on their cell surface which should be recognized (bound) by the antigen binding moiety of a target cell binding molecule as disclosed herein. The target cell may be a cancer cell or any other diseased cell.

The term "disorder" means a functional abnormality or disturbance in a subject such as a cancer, an autoimmune disorder, or an infection by virus, bacteria, parasite, or others.

The CARs as disclosed herein (polypeptide(s)), the nucleic acid molecule(s) encoding the CARs, recombinant expression vectors, cells expressing the CARs, and populations of cells expressing the CARs, and can be isolated and/or purified. The term "isolated" means altered or removed from the natural state. For example, an isolated population of cells means an enrichment of such cells and separation from other cells which are normally associated in their naturally occurring state with said isolated cells. An isolated population of cells means a population of substantially purified cells which are a more homogenous population of cells than found in nature. Preferably the enriched cell population comprises at least about 90% of the selected cell type. In particular aspects, the cell population comprises at least about 90%, 91%, 92%, 93%, 94%, 95%, 96%, 97%, 98%, 99%, or even 100% of the selected cell type. For example, a nucleic acid or a peptide naturally present in a living animal is not "isolated", but the same nucleic acid or peptide partially or completely separated from the coexisting materials of its natural state is "isolated". An isolated nucleic acid or protein can also exist in a non-native environment such as, for example, in a host cell.

As used herein, the term "subject" refers to a mammal such as mouse, rat, cow, pig, goat, chicken dog, monkey or human. Preferentially, the subject is a human. The subject may be a subject suffering from a disorder such as cancer (a patient), but the subject may be also a healthy subject.

The term "autologous" as used herein refers to any material derived from the same subject to who it is later re-introduced.

The term "allogeneic" as used herein refers to any material derived from a different subject of the same species as the subject to who the material is re-introduced.

The terms "therapeutically effective amount" or "therapeutically effective population" mean an amount of a cell population which provides a therapeutic benefit in a subject.

The terms "specifically binds" or "specific for" with respect to an antigen-binding domain of an antibody, of an antigen binding fragment thereof, as used e.g. in the TCBM as disclosed herein, or of a CAR refer to an antigen-binding domain which recognizes and binds to a specific antigen, but does not substantially recognize or bind other molecules in a sample. An antigen-binding domain that binds specifically to an antigen from one species may bind also to that antigen from another species. This cross-species reactivity is typical to many antibodies and therefore not contrary to the definition of that antigen-binding domain as specific. An antigen-binding domain that specifically binds to an antigen may bind also to different allelic forms of the antigen (allelic variants, splice variants, isoforms etc.) or homologous variants of this antigen from the same gene family. This cross reactivity is typical to many antibodies and therefore not contrary to the definition of that antigen-binding domain as specific.

The terms "engineered cell" and "genetically modified cell" as used herein can be used interchangeably. The terms mean containing and/or expressing a foreign gene or nucleic acid sequence which in turn modifies the genotype or phenotype of the cell or its progeny. Especially, the terms refer to the fact that cells, preferentially immune cells can be manipulated by recombinant methods well known in the art to express stably or transiently peptides or proteins which are not expressed in these cells in the natural state. For example, immune cells are engineered to express an artificial construct such as a chimeric antigen receptor on their cell surface. For example, the CAR sequences may be delivered into cells using a retroviral or lentiviral vector.

The terms "immune cell" or "immune effector cell" refer to a cell that may be part of the immune system and executes a particular effector function such as alpha-beta T cells, NK cells, NKT cells, B cells, innate lymphoid cells (ILC), cytokine induced killer (CIK) cells, lymphokine activated killer (LAK) cells, gamma-delta T cells, mesenchymal stem cells or mesenchymal stromal cells (MSC), monocytes or macrophages. Preferred immune cells are cells with cytotoxic effector function such as alpha-beta T cells, NK cells, NKT cells, ILC, CIK cells, LAK cells or gamma-delta T cells. "Effector function" means a specialized function of a cell, e.g. in a T cell an effector function may be cytolytic activity or helper activity including the secretion of cytokines.

The term "treat" (treatment of) a disorder as used herein means to reduce the frequency or severity of at least one sign or symptom of a disease or disorder experienced by a subject.

The term "expression" as used herein is defined as the transcription and/or translation of a particular nucleotide sequence driven by its promoter in a cell.

The term "epitope" means the part of an antigen that may be recognized by the immune system, specifically by antibodies, B cells, or T cells. For example, the epitope is the specific piece of the antigen to which an antibody or antigen binding fragment thereof binds.

The terms "linker/label epitope" (LLE) or "label/linker epitope" as used herein can be used interchangeably and refer to an epitope formed by the context, the interface and/or environment of conjugated linker moiety and label moiety of the TCBM as disclosed herein.

The epitope generated by the coupling of the label moiety with a linker moiety does not occur naturally in a subject. The generated epitope comprises a part of said LaM and a part of said LiM. Preferrentially, the LLE does not evoke or does not tend to evoke an immune reaction in a subject intended to be treated with cells expressing the CAR as disclosed herein. The only requirement for the LLE is that it is an epitope with regard to the CAR comprising the LLE binding domain. An extracellular LLE binding domain of a CAR as disclosed herein that is derived from an epitope recognizing molecule such as an antibody that recognizes the label/linker epitope binds with a higher preference to the newly created epitope, i.e. the label/linker epitope (the modified self antigen), than to the endogenous label moiety without linker moiety, i.e. the naturally occurring molecule in the subject (the self antigen).

Said extracellular LLE binding domain of said CAR binds with an at least twofold, preferentially at least 5-fold, more preferentially at least 10-fold higher affinity to said LLE than to the said naturally occurring molecule.

The amino acid sequences of SEQ ID NO:1 to SEQ ID NO:20 as given in the sequence listing protocol are mostly partial or full sequences of CARs as disclosed herein. Said sequences of SEQ ID NO:1 to SEQ ID NO:20 may also comprise variants of these sequences, respectively, which have some amino acids deleted, added or replaced while still retaining the intended function as described herein. Therefore, included in this definition are variants of the amino acid sequences in SEQ ID NO:1 to SEQ ID NO:20, respectively, such as amino acid sequences essentially similar to SEQ ID NO:1 to SEQ ID NO:20, respectively, having a sequence identity of at least 70%, or at least 75%, 80%, 85%, 90%, 95%, 97%, 98% or 99% at the amino acid sequence level. In general, all amino acid variations which do not lead to intentional changes of the intended function of the sequences SEQ ID NO:1 to SEQ ID NO:20, respectively, are included under this definition. In the context of the present invention, "sequence identity" may be determined using pairwise alignments using alignments programs for amino acid sequences well known to the art.

The "circulatory system" is an organ system of a subject that permits blood to circulate and transport nutrients (such as amino acids and electrolytes), oxygen, carbon dioxide, hormones, and blood cells to and from the cells in the body to provide nourishment and help in fighting diseases, stabilize temperature and pH, and maintain homeostasis. The circulatory system comprises two separate systems: the cardiovascular system, which distributes blood, and the lymphatic system, which circulates lymph.

The term "naturally occurring molecule in a subject or a derivate thereof' as used herein refers to molecules or substances in a subject, preferentially said molecules are located extracellularly or have at least an extracellular part, e.g. a transmembrane spanning protein. The naturally occurring molecule may exist in free form or covalently or non-covalently bound to another molecule, e.g. bound to a protein. For example, biotin exists in free form circulating in the blood system, but also bound to e.g. plasmaprotein.

Due to this requirement these molecules are non-immunogenic as they are endogenous molecules (self antigens) of the subject. As an example, biotin is a naturally occurring molecule in a subject as it is a circulatory molecule in the blood system (circulatory system) of a subject. The term "derivative thereof' means in this context that said naturally occurring molecule in a subject may undergo some minor modifications without changing the nature of said molecule. Said modifications are not identical with the conjugation of the linker moiety to said molecule.

### Embodiments

In addition to above described applications of the CAR system as disclosed herein, further embodiments of the invention are described in the following without intention to be limited to these embodiments.

In one embodiment of the invention the anti-LLE CAR is an anti- 6-[6-(biotinamido)hexanamido] hexanoyl moiety CAR, wherein said extracellular LLE binding domain comprises the sequence of SEQ ID NO: 1 and SEQ ID NO:2, and the TCBM comprises an 6-[6-(biotinamido)hexanamido] hexanoyl moiety and an ABM that binds specifically to an antigen of a cancer cell, e.g. anti-GD2 or CD19.

The results presented herein show that the CAR system of anti- 6-[6-(biotinamido)hexanamido] hexanoyl moiety CAR and TCBM comprising an 6-[6-(biotinamido)hexanamido] hexanoyl moiety work well regardless of the chosen ABM.

Several versions of anti- 6-[6-(biotinamido)hexanamido] hexanoyl moiety CAR structures with variable heavy-light chain orientations (HL and LH) of a scFV comprising SEQ ID NO: 1 and SEQ ID NO:2 and variable extracellular spacer sequences (FIG 2) have been generated and these sequences have been cloned into a polycistronic lentiviral vector backbone capable of encoding the CAR molecules as well as a marker (LNGFR).

FIG 3 shows that viral vector production and expression of LNGFR is possible with all constructs tested.

T cells where enriched from PBMCs, activated and transduced in vitro using the lentiviral vectors encoding the constructs indicated in FIG 2. The different anti- 6-[6-(biotinamido)hexanamido] hexanoyl moiety CAR T cells where then co-cultured with LS tumor cells at a ratio of 1 to 1. The anti-CD19 antibody conjugated with 6-[6-(biotinamido)hexanamido] hexanoyl moiety was added to the culture at a given dose or was not added (negative control). The cytotoxicity caused by the anti- 6-[6-(biotinamido)hexanamido] hexanoyl moiety CAR T cells was then measured. SEQ ID NO:11 proved to be the most effective construct (FIG 4).

Results were confirmed using a different tumor cell line and a different antibody (MCF7 and anti-CD19 respectively) as presented in FIG 5A-D.

A dose titration of the antibodies anti-GD2 or anti-CD19 showed that the anti- 6-[6-(biotinamido)hexanamido] hexanoyl moiety CAR T cells were effective at killing their tumor target specifically at doses as low as 1ng/ml (FIG 6A and B). This dose is considerably lower than doses required for antitumor functions in vivo. Cytotoxicity furthermore varied with different effector cell to target ratios (FIG 7A and B).

The anti- 6-[6-(biotinamido)hexanamido] hexanoyl moiety CAR T cells may not be cytotoxic against a tumor target in absence of labelled (e.g. biotinylated) antibodies or in the presence of non-labelled antibodies specific for the tumor or in presence of labelled antibodies not specific for the tumor. This is the case as shown in FIG 8 and FIG 9 using 2 different models. The anti- 6-[6-(biotinamido)hexanamido] hexanoyl moiety CAR function must not be impaired by potentially cross-reactive ligands present in the blood such as circulating biotin. Biotin is present in human serum in dose ranges from 60 to 370 pmol/L (Mock et al., 1995, J of Nutrition). It was shown that the function of the anti-6-[6-(biotinamido)hexanamido] hexanoyl moiety CAR is not impaired in presence of doses of biotin up to 30 µmοl/L (well in excess of physiological levels of biotin) or up to 50% human AB serum. See FIG 10 and FIG 11.

In another embodiment of the disclosure the anti-LLE CAR as disclosed herein is expressed in T cells from a donor (allogeneic) or a patient (autologous). These cells are activated and gene modified (using viral vectors or non-viral vectors) to express the anti-LLE CAR. These gene-modified T cells are infused into a patient as a therapeutically effective population of anti-LLE CAR expressing cells. The patient is infused a defined dose of a formulation of TCBM comprising an ABM directed against a target cell expressing the antigen recognized by the TCBM.

The TCBMs can be delivered to the recipient before, during and/or after infusion of anti-LLE CAR T cell infusion.

The anti-LLE CAR T cells will then recognize the TCBMs bound to their specific target. In turn the recognition of the bound TCBM by the anti-LLE CAR T cell induces the infused modified T cells to deliver their intended function - primarily that of expressing cytokines and or being cytotoxic (FIG 1).

For instance, the TCBMs may be targeting antigens at the surface of malignant cells enabling the anti-LLE CAR T cell to kill the tumor cells. In another application, the TCBMs infused target antigens expressed by cells infected with specific pathogens and enable the T cells to either mediate killing of the infected cells or release of inflammatory cytokines by the gene-modified T cells. In another instance the TCBMs bind to cell surface antigens or molecules expressed at sites of inflammation in order to induce a regulatory response from the T cells.

The difference in T cell response can be controlled by the type of T cells used (e.g cytotoxic CD8+ T cells or regulatory CD4+ T cells) and the type of intracellular signalling domains used to create the anti LLE CAR (e.g. CD3zeta and 4-1BB to induce proinflammatory responses or CD3zeta and PD-1 to induce anti-inflammatory responses).

In order to provide increased control and safety, one or combinations of formulations of TCBMs can be used in a dose, spatial and temporal manner.

A dose response of the TCBMs can be performed, whereby the patient receives the anti-LLE CAR T cells and a very low dose of TCBM followed by consecutive increasing doses of TCBM until the desired response is observed or until dose limiting toxicity is reached. Several TCBMs can be infused in order to target more than one target antigen expressed on one or more target cells effectively creating a polyclonal response.

TCBMs with different ABMs can be delivered one after the other.

Upon secession of TCBM delivery the anti-LLE CAR T cells are no longer capable of delivering their desired effector function. But this function can be resumed upon re-infusion of TCBM.

The CAR system as disclosed herein may also be used in the following way: The first wave of TCBM infused into the patient target immune modulatory cells (e.g Treg, M2, TAM, MDSC, myeloid derived cells, monocytes). And the consecutive waves of TCBMs target the tumor to be destroyed. This approach, aims to first destroy the immune barriers that prevent T cells from efficiently attacking the tumor only in a temporally restricted window of time.

In another embodiment of the disclosure, the anti-LLE CAR is expressed in NK cells and/or NKT cells which are very effective but short lived in vivo. This choice of cells as gene-modified effector cells is intended for treatments where the presence of gene-modified cells in vivo is only required over a defined period of time (e.g. less than 6 months, or even less than 2 months)

The anti-LLE CAR as disclosed herein may also be expressed in gamma/delta T cells in order to benefit from their effector, and homing capacity. This case is especially applicable in settings of allogeneic transfer from a donor to a recipient in order to avoid GvHD

In one embodiment of the disclosure the TCBM that bind to the anti-LLE CAR as disclosed herein may comprise two different antigen recognition sites in the ABM (herein referred to as "bispecific", instead of being bivalent for the same antigen) where each recognition site of the TCBM is of low affinity and/or high K_{off}, thus a target must express both antigens in order for the "divalent" TCBM to bind with an adequate avidity. Cells expressing only one target will not allow the "divalent" TCBM to bind sufficiently well for the anti-LLE CAR T cells to deliver their effector function.

In another approach the TCBM target peptide MHC complexes.

The anti-LLE CAR as disclosed herein may also be expressed in stem cells and/or multi-lineage progenitors and /or induced pluripotent stem cells that are preferably driven by their own promoter, and preferably a lineage specific promoter only active in T cells. Cells will then differentiate from the gene-modified stem cells and/or multi-lineage progenitors and give rise to T cells that will express the anti-LLE CAR as well as their own T cell receptor. This strategy is preferably used in cases where patients would undergo allo-stem cell transplantation. Once the stem cells and/or multi-lineage progenitors have given rise to T cells, the immune response can be driven towards the targeted disease by infusion of specific TCBM.

Alternatively, the T cells of a patient are directly modified in vivo using a modified viral vector capable of targeting specifically subsest of the adaptive immune system such as CD8 and / or CD 4 T cells, NK, NK T cells and/or gamma delta T cells

The anti-LLE CAR as disclosed herein may also be expressed in total T cells, CD8 positive T cells or CD4 positive T cells and used in combination with a library of antibodies and / or Fabs that are conjugated to a label/linker moiety as disclosed herein, e.g. 6-[6-(biotinamido)hexanamido] hexanoyl moiety to discover which combination of targeting motifs (e.g. antibodies) should be used to kill a given target cell. The appropriate combination may then be used to treat the disease of a given patient (i.e. guided combination CAR therapy).

In another embodiment of the disclosure T cells that express the anti-LLE CAR as disclosed herein and do not express TcRαβ are generated to create "universal-universal" (U2) CAR T cells. For example, stem cells that will be differentiated into T cells may be genetically engineered in this way, resulting in a complete set of modified T cells in the patient. The modified T cells may be given to a patient in form of an allo-transplantation. The U2 anti-LLE CAR approach meets the criteria for universal targeting since the specificity of the anti-LLE CAR T cell activation is absolutely determined by the predefined specificity of the TCBM. Moreover, the knock-out of the TcRαβ chain or CD3 with consecutive expression failure of the T cell receptor complex, facilitates the universal targeting in terms of donor universality with absence of GvHD guaranteed and further it facilitates the targeting of TcRαβ⁺ and CD3⁺ positive cells without self-destruction via CAR-targeting.

In another embodiment of the disclosure immune cells express the anti-LLE CAR as disclosed herein and in addition suppress or have been knocked out of accessory molecules that can be checkpoint, exhaustion or apoptosis associated signaling receptors as well as ligands such as PD-1, LAG-3, TIGIT, CTLA-4, FAS-L and FAS-R, moreover knockout of CD4 or CD8 in anti-LLE CAR cells would allow transient targeting of CD4+ or CD8+ cell which could be an attractive option for the treatment of autoimmune disease such as Graft versus Host disease, solid organ rejection, primary autoimmune disease: T and B cell associated autoimmunity (Rheumatoid arthritis, chronic inflammatory bowel diseases, multiple sclerosis, Myasthenia gravis and others).

As mentioned above the anti-LLE CAR as disclosed herein may be expressed in a cell such as an immune cell to target an antigen of a cancer cell for treating cancer. Cancers that may be treated include tumors that are not vascularized, or not yet substantially vascularized, as well as vascularized tumors. The cancers may comprise non-solid tumors (such as hematological tumors, for example, leukemias and lymphomas) or may comprise solid tumors. Types of cancers to be treated with the CAR system as disclosed herein include, but are not limited to, carcinoma, blastoma, and sarcoma, and certain leukemia or lymphoid malignancies, benign and malignant tumors, and malignancies e.g., sarcomas, carcinomas, and melanomas. Adult tumors/cancers and pediatric tumors/cancers are also included.

Examples of hematological cancers include leukemias, including acute leukemias (such as acute lymphocytic leukemia, acute myelocytic leukemia, acute myelogenous leukemia and myeloblasts, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors, such as sarcomas and carcinomas, include fibrosarcoma, myxosarcoma, liposarcoma, chondrosarcoma, osteosarcoma, and other sarcomas, synovioma, mesothelioma, Ewing's tumor, leiomyosarcoma, rhabdomyosarcoma, colon carcinoma, lymphoid malignancy, pancreatic cancer, breast cancer, lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, melanoma, and CNS tumors (such as a glioma (such as brainstem glioma and mixed gliomas), glioblastoma (also known as glioblastoma multiforme) astrocytoma, CNS lymphoma, germinoma, medulloblastoma, Schwannoma craniopharyogioma, ependymoma, pinealoma, hemangioblastoma, acoustic neuroma, oligodendroglioma, menangioma, neuroblastoma, retinoblastoma and brain metastases).

The anti-LLE CAR as disclosed herein may be expressed in an immune cell to target an antigen associated with a virus, bacteria, parasite, or other infection in order to treat the infection.

The anti-LLE CAR as disclosed herein may be expressed in cell such as an immune cell to target a self antigen to treat an autoimmune disorder.

However, the CAR system as disclosed herein should not be construed to be limited solely to the antigen targets and diseases disclosed herein. Rather, it should be construed to include any antigenic target that is associated with a disorder where a CAR system as disclosed herein may be used to treat the disorder.

### Examples

### Example 1. Generation of anti-LLE CAR T cells

### 1.1 Generation of a library of lentiviral transfervector constructs encoding different spacer variants of the anti-LLE-CAR

In order to generate anti-LLE CARs recognizing biotin, as well as parts of the linker moiety connecting biotin to antibody recognizing the target cell epitope, an scFv (single chain variable fragment) was designed based on the variable heavy (VH) and variable light (VL) chains of the anti-bioMB antibody (SEQ ID NO:1 and 2) in VH-VL and VL-VH orientation. Variable chains were connected by a flexible (G₄S)₃ linker (SEQ ID NO: 4) and the human CD8 leader sequence (SEQ ID NO:3) was placed N-terminally of the scFv. Sequences were optimized for expression in human host cells and synthesized by DNA2.0 with BamHI and NheI cut sites flanking 5' and 3' of the scFv sequence, respectively. These scFvs were subcloned into transfervector plasmids containing different spacer variants based on human IgG4 (HC) and human CD8 stalk, a 2A element as well as an expression marker (LNGFR) (FIG 2). Plasmid DNA was amplified in E.coli NEB5alpha and prepared using the Qiagen EndoFree Plasmid Maxi Kit according to the manufacturer's protocol. All sequences were verified by Sanger sequencing (GATC).

### 1.2 Generation of LV particles

For production of lentiviral particles 0,5 x 10⁵/cm² HEK293T cells were seeded in a T175 cell culture flask (175cm²) using 25 mL medium (DMEM, 10% (v/v) FCS, 2 mM L-glutamine) and incubated overnight at 37°C, 5% CO₂. On the next day the adherent cells typically showed 75-90% confluency. Medium was exchanged to DMEM with 2 mM L-glutamine, without FCS and transfection DNA mixes were prepared (using Lipofectamine 3000, ThermoFisher reagent protocol). For this a total DNA amount of 0,2 µg / cm² (35 µg for one T175 flask) was used and two separate mixes A (DNA) and B (Lipofectamine) were prepared. For Mix A: Transfer-vector-plasmid 0,073 µg / cm² (12,7 µg), helper-plasmid 1 (VSVg) 0,018 µg / cm² (3,18 µg), helper plasmid 2 (gag/rev/pol) 0,109 µg / cm² (19,09 µg), P3000 enhancer reagent 2 µL/µg total DNA, and Opti-MEM 0,033 mL / cm² were mixed by brief vortexing. For Mix B: Lipofectamine 3000 0,75 µL / cm² was resuspended in Opti-MEM 0,033 mL / cm². Mix A and B were incubated separately for 5 minutes, then mix A and B were mixed together gently, and incubated 20 minutes at RT. Then the lipid/DNA complexes were added dropwise to plated cells and incubated at 37° C for approximately 12 hours. After this, the medium was replaced with 0,133 mL/cm² fresh medium (DMEM, 10% FCS, 2mM L-glutamine) and incubated at 37° C, 5% CO2 for 24 hours. Assembled lentiviral particles were harvested by carefully removing the cell culture supernatant by pipetting. Cellular debris was removed from the supernatant by centrifugation for 10 min at 5000*xg*. The LV-containing supernatant was then directly used for transduction of T cells, stored overnight at 4°C or snap frozen in liquid nitrogen and stored at -80°C. For a second round of lentiviral particle production 0,133 mL/cm² fresh medium (DMEM, 10% FCS, 2mM L-glutamine) was added to the transfected HEK293T cells and incubation was continued for another 24h at 37°C, 5% CO₂. After 24 h the supernatant was harvested as described previously.

### 1.3 Transduction, expansion, and expression analysis of anti-LLE CAR T cells

Blood samples were drawn from healthy donors and PBMCs were generated by separation on Biocoll (Biochrom). CD4⁺ and T cells were selected using MACS technology (Miltenyi Biotec). For T cell activation and -expansion the T cell activation/expansion kit, human (Miltenyi Biotec) was used according to the manufacturer's instructions. T cells were seeded into 24-well plates with 2 mL T cell suspension per well at a concentration of 1x10⁶ cells/mL in TexMACS medium containing human AB serum (10% (v/v) GemCell), IL-7 (10 ng/mL) and IL-15 (5 ng/mL).

Transduction was performed on day 2 after activation. For this, LV supernatant was added to the T cells at a MOI of 3 and cells were carefully resuspended by pipetting up and down. On day 8 after activation, LNGFR-expressing cells were separated by MACS using anti-LNGFR microbeads on an LS column (Miltenyi Biotec) according to the manufacturer's instructions. Selected cells were reactivated using the T cell activation/expansion kit, human (Miltenyi Biotec) and seeded in a 24-well plate at a concentration of 1x10⁶ cells/mL (2 mL per well). Cells were then continuously expanded and transduction efficiency was determined on day 14 after their first activation by FACS analysis. For this an aliquot was removed and stained for LNGFR expression using anti-LNGFR APC (Miltenyi Biotec) according to the manufacturer's instructions (FIG 3).

### Example 2. TCBM generation

Antibody modification by random labeling by succinimidyl-esters at amino groups was done according to protocols well known in the art. For modification, antibodies were rebuffered into PEB buffer by passing over an Sephadex G25 column equilibrated in buffer. Collected fractions were assayed for protein content using the Bradford assay. Protein containing fractions were pooled and the total volume determined. Final protein concentration was measured by absorption at 280nm. Subsequently, the corresponding amount of Biotin-LC-LC-NHS (ThermoFisher Scientific, Mw 567,70 g/mol, Cat. No. 21343, CAS-No. 89889-52-1) was dissolved in DMSO. The antibody and DMSO/label mix was incubated at room temperature (21°C) for 1h and then passed over a Sephadex G25 column. Again fractions were collected, assayed for protein concentration and protein containing fractions were pooled. Final protein concentration was measured by absorption at 280nm. Successful biotinylation was confirmed by incubation of the antibodies cell line expressing the antibody target and secondary staining with a fluorochrome conjugated anti-biotin antibody, followed by FACS analysis. Furthermore, the HABA assay was used for determination of number of biotin molecules per antibody.

### Example 3. Generation of target cell lines for cytotoxicity analysis

### 3.1 Generation of stably transduced luciferase expressing cell lines

Generation of LV particles was similar according to 1.2. The tumor cell transduction protocol was similar described to that for T cells (described under 1.3). The used transfer-vector-plasmid pCDH-EF-eFFly-T2A-mCherry and pCDH-EF-eFFly-T2A-GFP was generously provided by the Helmholtz Zentrum München (German Research Center for Environmental Health, by the department of gene vectors, Dr. Binje Vick and PD Dr. med. Irmela Jeremias). After transduction, tumor cells were upscaled and transduction efficiency was evaluated by flow cytometry (GFP excitation 488 nm laser, band pass filter 525/50 nm; mCherry excitation 561 nm laser, band pass filter 615/20 nm). Cells were enriched by FACS-cell sorting for the medium-high (GFP/mCherry) population, negative, low-positive and high-positive cell subsets were discarded. Medium-high (GFP/mCherry) tumor cell lines were upscaled again, evaluated for (GFP/mCherry) fluorescence, cryopreserved and used for functional analyses. The following cell lines were stably transduced with pCDH-EF-eFFly-T2A-mCherry and/or pCDH-EF-eFFly-T2A-GFP: NALM-6 and NALM-16 which are both B cell precursor leukemia cell lines, TcPγδ positive MOLT14 and TcRαβ positive JURKAT which are both T cell acute lymphoblastic leukemia cell lines, as well as LS and LAN-1 which are both Neuroblastoma cell lines.

### 3.2 Generation of MCF-7 transfectant expressing truncated CD19

Adherent mammary carcinoma cell line MCF-7 (Michigan Cancer Foundation - 7) cell line was stably transfected by electroporation in IMDM-media (Iscove's Modified Dulbecco's Medium, ThermoFisher Scientific) on ice by transfecting linearized truncated CD19 DNAconstructs. Electroporation was done at a voltage of 200 V and 975 µF with a time constant between 40 to 80. For selection antibiotic G418 (Geneticin, InvivoGen) was used to at 1 mg/mL. Via FACS-based cell sorting homogenously expressing MCF-7 with medium-high tCD19 expression were isolated and upscaled for cryopreservation and functional analysis.

### Example 4. xCELLigence Real Time Cell Analysis (RTCA) device based-kill assay

### 4.1 LS cultivation for xCELLigence

- 3 days of cultivation (optimal)
- 5866 cells/cm2 -> middle cell culture bottle (75 cm2) adjusted to 0,44x10e6 cells in 15mL medium or big cell culture bottle (175 cm2) adjusted to 1x10e6/mL in 35 mL medium.
- Use cells only at a confluency of 60 and 80%

### Harvesting and seeding:

a) Discard cultivation media (RPMI 1640, 10% FCS, 1% Penicillin/Streptomycin, 5% L-glutamine)
b) Add 3 mL Trypsin/EDTA per T175 cm2 flask and incubate for 2 minutes
c) Add 8 mL for complete media, carefully dispense cells and centrifuge at 300 g for 5 minutes
d) Take cells into 10 mL fresh media and count cells with trypan blue solution
e) Seed cells according to xCELLigence protocol

### 4.2 xCELLigence kill assay protocol

### Day 0:

- ExpNotes: name experiment and save it under the designation administrator (Data directory)
- Layout: name wells
- Schedule: add step 1 (once) for calibration (adjustment automatically), add step2 (interval 30min (15minutes), complete measurement time 48h (100h)

### Calibration of RTCA device:

- Fill in media 50µL/well, put the plate into the measurement device and press the start button to start calibration. Take out plate when finished measurement ("Ready for next step") Targets:
- Adjust LS (cultivated for three days before) to 0,15 x 10E6/mL
- seed tumor cells at 15000 cells/well in 100 µL
- add 100 µL of media/well
- Put the plate back into the measurement device and press the start button
- Note starting time

### Day 1:

After 24 hours the cell index is between 1-3. Press the stop button and take the plate out of the measurement device in order to continue the experiment. If cell index is below 1, discard experiment.

Pipette within 30 minutes:
a) Carefully take out 100 µL of media (remaining volume is 100 µL)
b) Add effector cells in 50 µL volume
c) Add mAb in 50 µL volume
d) Total volume in all wells: 200 µL (fill up with media in without-mAb condition)

Place plate in the measurement device and continue data acquisition.

### Obligatory controls:

- Background: media only per 4 wells +/- mAb
- Target only per 4 wells +/- mAb

### Example 5. Luciferase based-kill assay

### 5.1 Background

D-luciferin is the substrate for firefly luciferase's bioluminescence reaction. It linearly correlates with viability and inversely correlates with target cell death.

### 5.2 Preparation of D-Luciferin-aliquots

a) Dissolve 10 mg D-Luciferin (synthetic, Sigma Aldrich, Cat.No: L9504 10MG) in 1 mL of DMSO, to attain the concentration of 10 mg/mL
b) Add 24 mL of DMSO → concentration of 0.4 mg/mL
c) Prepare 55 µL aliquots (10% extra volume)
d) Store at -20°C

### 5.2.1 Usage

a) Take 50 µL from 55 µL aliquot and add 5 mL of media used in the experiment (CAVE: for human plasma experiments use RPMI only, w/o FCS) → 1:100 dilution → concentration of 0.004 µg/µL = 4µg/mL
b) if pipetting volume is 50 µL and final volume in each well is 200 µL → 1:4 dilution, final concentration is 1 µg/mL

### 5.3 Materials, Cells, Reagents, and Instruments

a) Stably transduced firefly luciferase positive target cell lines
b) Effector cells
c) RPMI Medium, Merck-Millipore, Cat no: F1215-500mL
d) Flat bottom white plates, Greiner bio one, Cat.No: 655083
e) D-luciferin synthetic, Cat.No.: L9504, Sigma Aldrich
f) ADVIA 120 Siemens Bayer (cell counting)
g) Perkin Elmer Wallac 1420 Victor2 Microplate Reader (plate reader)

### 5.4 Procedure

### a) Target cells preparation: (Calculations for one plate)

Target cells are counted with the ADVIA for the assay. Take 10E06 cells from the cell stock, centrifuge at 300 g for 10 minutes, remove supernatant completely and adjust the cell number for 0.1^{∗}10E6 cells/50 µL.

In order to guarantee low well per well deviation, column 1 and 12 are used as 100% control, tumor cells only. Moreover, the bioluminescence titration 100%, 75%, 50%, 25%, 10% and 0% of target cells is used to calculate the lysis formula in Excel by linear trendline calculation.

### b) Effector cell preparation

Effector cells are counted with the ADVIA and added in 50 µL media.
c) Antibody is added in the corresponding condition at predefined concentrations in 50 µL media.
d) Add D-luciferin according to the "Preparation of the D-luciferin aliquots"
e) Place the plates in the incubator at 37°C, 5% CO2.
f) Measure bioluminescence at 6, 12, 24 and 48 hours.
g) Instrument setting: Acquisition of bioluminescence, 37°C, VICTOR Wallac reader

### Example 6. Intracellular Cytokine staining (ICS)

### 6.1 Buffer/reagents

- PBS-EDTA: 500mL PBS, 2mM EDTA (2mL when you have a concentration of 5M)
- FACS-buffer: 500mL PBS, 2%FCS (10mL), 2mM EDTA (2mL when you have a concentration of 5M), 0,02% NaAzid (1 mL when you have a concentration of 10%)
- PermWash: 500mL PBS, 0,1% Saponin-S7900 (0,5g), 0,05%BSA A-3059 (2,5g), 0,02% NaAzid (1 mL when you have a concentration of 10%)
- Brefeldin A Sigma (1:125) AND/OR Monensin, for example GolgiStop
- Brefeldin is suggested for intracellular cytokine staining, Monensin for CD107a
- Live/Dead coloring: Invitrogen
- Cytofix/Cytoperm: BD
- Antibodies of interest
- 96 well round bottom plate

### 6.2 Procedure

- Use every second well to avoid contamination during the washing procedure
- Total incubation volume 200 µL
- Incubation time 16 hours

Starting the experiment: Add 50 µL of Brefeldin A in each well.

Needed for compensation: Unstained cells (exactly the same used in the experiment). They are treated in the same way during the assay, but are not stained.

### Staining 1-3

Preparation 30 minutes before starting:
- Cool down the centrifuge to 4°C
- Prepare buffers
- Get ice to store plates on ice
- Produce the live/dead staining solution: 50 µL/well, Aqua 1:400

### After incubation time

- centrifuge plate (1800 rpm, 2min, 4°C)
- wash with 200 µL PBSE (1800 rpm, 2min, 4°C)
- wash with 200 µL PBSE (1800 rpm, 2min, 4°C)
   a) Add 50 µL/well live/dead solution and resuspend cells (not unstained condition) Incubation time 20 minutes at 4°C (dark)
      During this time: Produce the extracellular mastermix (in FACS-buffer), all together in one falcon
      After incubation time of L/D solution:
      - Add 150µL FACS-buffer (1800 rpm, 2min, 4°C)
      - Washing with 200µL FACS buffer (1800 rpm, 2 min, 4°C)
   b) Add 50 µL/well extracellular mastermix and resuspend cells (neither unstained, nor isotype condition)
      Incubation time 20 minutes at 4°C (dark)
      After incubation time:
      - Add 150 µL FACS-buffer (1800 rpm, 2 min, 4°C)
      - Washing with 200 µL FACS buffer (1800 rpm, 2 min, 4°C)
      - Add 100 µL/well Cytofix/Cytoperm and resuspend it
      Incubation time 20 minutes at 4°C (dark)
      During this time: Produce the intracellular mastermix (in PermWash), all together in one falcon
      After incubation time (Cytofix/Cytoperm):
      - Add 100µL/well PermWash (1800 rpm, 2 min, 4°C)
      - Washing with 200µL PermWash (1800 rpm, 2 min, 4°C)
   c) Add 50 µL/well intracellular Mastermix and resuspend it (not unstained)
      Incubation time 20 minutes at 4°C (dark)
      During this time: Produce PFA solution (always a new one, don't keep it), FACS-buffer + 1% paraformaldehyde, stock concentration 37%, 200 µL/well
      After incubation time (IZ):
      - Add 150 µL PermWash (1800 rpm, 4°C, 2 min)
      - Washing with 200 µL PermWash (1800 rpm, 2 min, 4°C)
      - Washing with 200 µL PermWash (1800 rpm, 2 min, 4°C)
      - Add 200 µL/well PFA and resuspend it
      Store at 4-8°C until FACS analysis

### Example 7. Generation of anti-LLE CAR⁺ TcRαβ⁻ T cells using the CRISPR/Cas9 technology

*Anti-LLE CAR*⁺ *TcRαβ⁻* cells were generated from anti-LLE CART cells (SEQ ID NO:11), according to the protocol presented above. The knock-out of the TcRαβ chain was accomplished by mRNA based CRISPR/Cas9 technology using the Neon@ Transfection System (electroporation). Highly purified anti-LLE CART cells were enriched by MACS technology for the generation of U2 anti-LLE CART cells.

### 7.1 Electroporation of CAR T cells using Neon® Transfection System

### Materials/Technology:

a) Anti-LLE CART cells (SEQ ID NO:11) were generated as presented in 1.3.
b) CART cell cultivation media (TexMACS, 10% human serum, 10 ng/mL IL7, 5 ng/mL IL15)
c) DPBS - Dulbecco's Phosphate-Buffered Saline
*d)* Custom modified gRNA (guide RNAs sequences for TCR knockdown have been described in literature and should be accessible to the person skilled in the art)
e) Cas9 mRNA was ordered from tebu-bio, Cat.no.: L-6125-100
f) Neon@ Transfection System 10 µL Kit, ThermoFisher Scientific, Cat.no.: MPK1025
- Electrolytic buffer E
- Resuspension buffer T
- 10 µL Neon@ tips
- Neon@ electroporation tubes

| | Stock concentration | per 1E06 cells used amount |
|---|---|---|
| Cas9 mRNA | 1000 ng/µL | 5 µg |
| sgRNA | 5000 ng/µL | 25 µg |

### Procedure:

a) Transfer 2E06 CAR T cells (SEQ ID NO :11) in microcentrifuge tubes
b) Wash CAR T cells (SEQ ID NO: 11) cells 3 times with 1 mL PBS buffer in microcentrifuge at 800 g, 5 min and remove supernatant carefully

Use reagents as listed:

| | Control w/o sgRNA | with sgRNA |
|---|---|---|
| Buffer T | 100 µL | 90 µL |
| Cas9 mRNA | 10 µL | 10 µL |
| sgRNA | 0 µL | 10 µL |
| Total volume | 110 µL | 110 µL |

a) Fill electroporation tube with buffer E (3 mL)
b) Load program
c) Use 100 µL electroporation tip and take 100 µL of the corresponding sample for each electroporation
d) Calibrate electroporation device Neon using 100 µL buffer T
e) Continue with sample electroporation if self-check is approved OK, without error Electroporation settings: Voltage 1.400, Pulse with 10, Pulse number 3, Tip type 100 µL
f) For each electroporation, put the electroporation pipette into the electroporation tube
g) Start electroporation process
h) After electroporation, immediately remove the Neon@ Pipette and transfer the samples from the 10 µL Neon@ Tip into prewarmed 900 µL CAR T cell culture medium.

Detection of successful knock-out is performed by flow cytometry and T7 test (Example 8).

### Example 8. Functional analysis of anti-LLE CAR modified T cells

T cells where enriched from PBMCs, activated and transduced *in vitro* using the lentiviral vectors encoding the constructs indicated. The different anti-LLE CAR T cells where then co-cultured with tumor cells (NALM6) at a ratio of 1 to 1. The biotinylated anti-CD 19 antibody was added to the culture at a given dose or was not added (negative control). The cytotoxicity caused by the anti-LLE CAR T cells and CD19 CAR T cells was then measured. (FIG 4). The CAR T cells expressing the CAR encoded by SEQ ID NO:11 showed the greatest cytotoxic effect, comparable to direct anti-CD19 CAR T cells (positive control). Results represent the mean of four independent experiments performed in triplicates from four different donors. Results were confirmed using a real-time cytotoxicity assay (RTCA xCELLigence) and different tumor cell lines. Anti-LLE CAR T cells exhibited cytotoxcicity against LS cells in presence of biotinylated anti-GD2 antibody (FIG 5B) again with the CAR encoded by SEQ ID NO: 11 giving the most potent cytotoxic response. Furthermore anti-LLE CAR T cells exhibited cytotoxcicity against MCF-tCD19 cells in presence of biotinylated anti-CD 19 antibody (FIG 5D). The mAb titration for different tumor associated antigens e.g. CD19 and GD2 showed favorable anti-LLE CAR T activity in the range of 100 pg/mL-10 µg/mL target specific tagged mAb concentration (FIG 6A and B). This dose is considerably lower than the antibody doses required for effective therapeutic application *in vivo.* In the clinical trial "CH14.18 1021 Antibody and IL2 After Haplo SCT in Children with Relapsed Neuroblastoma" registered at clinicaltrials.gov (NCT02258815) the anti-GD2 mAb CH14.18 is dosed with 20 mg/m² in five consecutive days, resulting in high mAb serum-concentrations above 20 µg/mL (unpublished data, study recruiting). Accordingly, high mAb concentrations are being observed in our patient-individual compassionate use with the Fc-optimized chimeric anti-CD19 mAb treatment (4G7SDIE) in high-risk B-lineage precursor ALL patients. 4G7SDIE is applied with 20 mg/m² every 2 weeks. Indeed, after infusion of anti-CD19 antibodies *in vivo,* it has been reported that levels of 60-90 µg/mL can be detected in the blood (MolTher.2016, PMID: 27380762). An effector to target titration showed a linear correlation (FIG 7A and B).

Furthermore, anti-LLE CAR T cells exhibited low cytotoxicity against tumor target cells in absence of tagged antibodies or in the presence of non-tagged antibodies specific for the tumor or in presence of tagged antibodies not specific for the tumor, using two different models (FIG 8, FIG 9). xCELLigence RTCA was performed as outlined in 4 with the neuroblastoma cell line LS (GD2 expressing tumor cell line) at an effector to target ratio of 1:1. In this experiment only slight differences were observed for the conditions tumor cell line only without effector cells (anti-LLE CAR T cells), with anti-LLE CAR T cells without mAb as well as in the conditions anti-LLE CAR T cells with non-tagged GD2-specific mAb CH14.18 and anti-LLE CARTs specific tagged mAb (4G7SDIE) binding to CD19, which is not expressed by LS (FIG 8). Whereas complete lysis of LS was observed only in the condition anti-LLE CARTs plus tagged GD2-specific mAb CH14.18 within less than 24 hours. After 24 h some minor unspecific anti-tumor activity cannot be excluded from the anti-LLE CART cells (FIG 8). Similar results for preferential lysis of NALM6 target cells by anti-LLE CAR T cells only in presence of anti-CD19-biotin (4G7-Biotin), were obtained in a luciferase based assay (FIG 9).

Finally, the anti-LLE CAR function must not be impaired by potentially cross-reactive ligands present in the blood such as circulating biotin. It was shown that the function of the anti-LLE CAR is not impaired in presence of doses of biotin up to 30 µg/mL (well in excess of physiological levels of biotin) or up to 50% human AB serum (FIG 10 and 11). xCELLigence RTCA was performed as outlined in 4. with the neuroblastoma cell line LS (GD2 expressing tumor cell line) and anti-LLE CAR T cells at an effector to target ratio of 1:1 in the absence or presence of free biotin, which was added at low (physiological) to supraphysiological concentrations in the cell culture media. Irrespective of free biotin concentration, a complete eradication of tumor cells was observed in less than 24 h in the testing condition with anti-LLE CAR T cells and 10 ng/mL GD2-specific mAb CH14.18 (FIG 10). Moreover, no inhibition of anti-LLE CAR T cells was observed during cytotoxicity measurements performed with the B-cell precursor leukemia cell line NALM-6 (FIG 11). Neither free biotin at physiological and supraphysiological concentrations nor human AB serum) decreased specific lysis of anti-LLE CART cell mediated lysis (FIG 11).

In order to generate "universal-universal" (U2) CAR T cells, anti LLE-CAR T (SEQ ID NO:11) TCRαβ knock cells were generated as described in Example 7. Successful knock out was confirmed by TCRαβ expression analysis (FIG 12A and B). Flow cytometry was done according to common staining protocols. Cells washed twice in PBS human albumin containing buffer, centrifuged at 300g for 10 min (acceleration/brake 9/9), stained for 15 minutes in the dark at 4°C, then washed again twice before acquisition on the BD-LSR II according to manufacturer's protocol after compensation with anti-mouse compensation beads (BD Biosciences, ordering No. 552843) was performed according to manufacturer's protocol. TcRab FITC (Miltenyi Biotec, Anti-TCRa/p-FITC, anti-human (clone: BW242/412), ordering No. 130-098-690), LNGFR APC (Miltenyi Biotec, CD271 (LNGFR)-APC, anti-human (clone: ME20.4-1.H4), ordering No. 130-091-884), CD8 APC-Cy7 (BioLegend, anti-human (clone: SK1), ordering No. 344713), CD4 UV1 BUV 395 (BD Biosciences, (clone: RPA-T4) ordering No. 564724) were used. While the CD4/CD8 ratio remains unchanged, a reduction in TcRαβ expression is clearly visible (FIG 12 A). This result was confirmed by T7 and TIDE which was performed according to protocols well-known in the art (FIG 12 B). Impedance based real-time cytotoxicity assay (RTCA xCELLigence) of anti-LLE CAR T cells (SEQ ID NO:11) and TcRαβ knockout anti-LLE CAR T cells (SEQ ID NO:11) versus LS was performed in presence and absence of lOng/ml anti-GD2-biotin (ch14.18-biotin) antibody, which showed that TcPαβ knockout anti-LLE CAR (SEQ ID NO:11) T cells generated by CRISPR/Cas9 technology perform equally well in vitro cytolysis as TcRαβ positive anti-LLE CAR (SEQ ID NO:11) T cells (FIG 12 C). Also during specifc lysis of MOLT14 target cells by anti-LLE CAR T cells (SEQ ID NO:11) and TcRαβ knockout anti-LLE CAR T cells (SEQ ID NO:11), similar target lysis was observed by anti LLE CAR T cells expressing a TcRαβ chain or not (FIG 12 D). Furthermore, TcRαβ knockout anti-LLE CAR T cells, significantly decreased survival of JURKAT target cells when incubated for 48h in presence of 10 ng/ml anti-TcRαβ-biotin mAB, at an effector : target cell ratio of 1:1 (FIG 12 E)

## Claims

1. A composition comprising
a) an anti-linker/label epitope chimeric antigen receptor (anti-LLE CAR) comprising
I) an extracellular linker/label epitope (LLE) binding domain,
II) a transmembrane domain, and
III) a signal transduction domain,
wherein said extracellular LLE binding domain binds a target cell binding molecule (TCBM),
b) said target cell binding molecule (TCBM) comprising
i) an antigen binding moiety (ABM), wherein said ABM binds specifically to an antigen expressed on a target cell,
ii) a label moiety (LaM), wherein said LaM is a naturally occurring molecule in a subject or a derivative thereof, wherein said naturally occurring molecule is in the circulatory system of said subject,
iii) a linker moiety (LiM) conjugating said ABM and said LaM, thereby forming a linker/label epitope (LLE), wherein said LiM is selected from the group consisting of peptides, alkyl chains, alkanoic acids, carboxylic acids, farnesyls, polyethylene glycols, or a derivative thereof,
wherein said extracellular LLE binding domain binds said LLE with a higher preference than said naturally occurring molecule, wherein said extracellular LLE binding domain binds with an at least 10-fold higher affinity to said LLE than to said naturally occurring molecule.

2. The composition according to claim 1, wherein the k(off) value for the binding between said extracellular LLE binding domain is higher to a monomeric LLE and said naturally occurring molecule than to a multimeric LLE.

3. The composition according to claim 1 or 2, wherein said LLE is generated site-specifically, thereby forming an epitope comprising a part of said LaM and a part of said LiM.

4. The composition according to any one of claims 1 to 3, wherein said LaM is selected from the group consisting of amino acids, peptides, creatinine, biotin, biocytin, hormones, vitamins, or a derivative thereof.

5. The composition according to any one of claims 1 to 4, wherein said LiM is a molecule capable of generating said LLE.

6. The composition according to any one of claims 1 to 5, wherein said LaM is biotin or a derivative thereof and said LiM is a 6-(6-aminohexanamido) hexanoyl moiety or a 6-aminohexanoyl moiety.

7. The composition according to any one of claims 1 to 6, wherein said extracellular LLE binding domain comprises the sequence of SEQ ID NO: 1 and SEQ ID NO:2.

8. The composition according to any one of claims 1 to 7, wherein said antigen that is bound by said ABM is expressed on the surface of a cancer cell.

9. The composition according to any one of claims 1 to 8, wherein said antigen is selected from the group consisting of CD33 (Siglec-3), CD123 (IL3RA), CD135 (FLT-3), CD44 (HCAM), CD44V6, CD47, CD184 (CXCR4), CLEC12A (CLL1), LeY, FRβ, MICA/B, CD305 (LAIR-1), CD366 (TIM-3), CD96 (TACTILE), CD133, CD56, CD29 (ITGB1), CD44 (HCAM), CD47 (IAP), CD66 (CEA), CD112 (Nectin2), CD117 (c-Kit), CD133, CD146 (MCAM), CD155 (PVR), CD171 (L1CAM), CD221 (IGF1), CD227 (MUC1), CD243 (MRD1), CD246 (ALK), CD271 (LNGFR), CD19, CD20, GD2, and EGFR.

10. The composition according to any one of claims 1 to 9, wherein said ABM is an antibody or an antigen-binding fragment thereof.

11. The composition according to any one of claims 1 to 10, wherein said anti-LLE CAR comprises the sequence of SEQ ID NO: 11, and wherein said extracellular LLE binding domain binds to a 6-[6-(biotinamido)hexanamido] hexanoyl moiety.

12. The composition according to any one of claims 1 to 11, wherein said anti-LLE CAR is expressed in a T cell, NK cell, NKT cell, gamma/delta T cell, Treg cell, or stem cell.

## Patentansprüche

1. Zusammensetzungen, umfassend
a) einen chimären Anti-Linker/Label-Epitop-Antigenrezeptor (Anti-LLE CAR), umfassend
I) eine extrazelluläre Linker/Label-Epitop(LLE)-Bindungsdomäne,
II) eine Transmembrandomäne, und
III) eine Signaltransduktionsdomäne,
wobei die extrazelluläre LLE-Bindungsdomäne ein Zielzell-Bindungsmolekül (TCBM) bindet,
b) wobei das Zielzell-Bindungsmolekül (TCBM) umfasst
i) eine Antigenbindungseinheit (ABM), wobei die ABM spezifisch an ein Antigen bindet, das auf einer Zielzelle exprimiert wird,
ii) eine Labeleinheit (LaM), wobei die LaM ein natürlich vorkommendes Molekül in einem Subjekt oder ein Derivat davon ist, wobei sich das natürlich vorkommende Molekül im Kreislaufsystem des Subjekts befindet,
iii) eine Linkereinheit (LiM), die die ABM und die LaM konjugiert, wodurch ein Linker/Label-Epitop (LLE) gebildet wird, wobei die LiM ausgewählt ist aus der Gruppe bestehend aus Peptiden, Alkylketten, Alkansäuren, Carbonsäuren, Farnesylen, Polyethylenglykolen oder einem Derivat davon,
wobei die extrazelluläre LLE-Bindungsdomäne das LLE mit einer höheren Präferenz bindet als das natürlich vorkommende Molekül, wobei die extrazelluläre LLE-Bindungsdomäne mit einer mindestens 10-fach höheren Affinität an das LLE bindet als an das natürlich vorkommende Molekül.

2. Zusammensetzungen nach Anspruch 1, wobei der k(off)-Wert für die Bindung zwischen der extrazellulären LLE-Bindungsdomäne an ein monomeres LLE und das natürlich vorkommende Molekül höher ist als an ein multimeres LLE.

3. Zusammensetzungen nach Anspruch 1 oder 2, wobei das LLE ortsspezifisch erzeugt wird, wodurch ein Epitop gebildet wird, das einen Teil der LaM und einen Teil der LiM umfasst.

4. Zusammensetzung nach einem der Ansprüche 1 bis 3, wobei die LaM ausgewählt ist aus der Gruppe bestehend aus Aminosäuren, Peptiden, Kreatinin, Biotin, Biocytin, Hormonen, Vitaminen oder einem Derivat davon.

5. Zusammensetzung nach einem der Ansprüche 1 bis 4, wobei die LiM ein Molekül ist, das in der Lage ist, das LLE zu erzeugen.

6. Zusammensetzung nach einem der Ansprüche 1 bis 5, wobei die LaM Biotin oder ein Derivat davon ist und die LiM eine 6-(6-Aminohexanamido)hexanoyl-Einheit oder eine 6-Aminohexanoyl-Einheit ist.

7. Zusammensetzung nach einem der Ansprüche 1 bis 6, wobei die extrazelluläre LLE-Bindungsdomäne die Sequenz von SEQ ID NO: 1 und SEQ ID NO: 2 umfasst.

8. Zusammensetzung nach einem der Ansprüche 1 bis 7, wobei das Antigen, das durch die ABM gebunden wird, auf der Oberfläche einer Krebszelle exprimiert wird.

9. Zusammensetzung nach einem der Ansprüche 1 bis 8, wobei das Antigen ausgewählt ist aus der Gruppe bestehend aus CD33 (Siglec-3), CD123 (IL3RA), CD135 (FLT-3), CD44 (HCAM), CD44V6, CD47, CD184 (CXCR4), CLEC12A (CLL1), LeY, FRβ, MICA/B, CD305 (LAIR1), CD366 (TIM-3), CD96 (TACTILE), CD133, CD56, CD29 (ITGB1), CD44 (HCAM), CD47 (IAP), CD66 (CEA), CD112 (Nectin2), CD117 (c-Kit), CD133, CD146 (MCAM), CD155 (PVR), CD171 (L1CAM), CD221 (IGF1), CD227 (MUC1), CD243 (MRD1), CD246 (ALK), CD271 (LNGFR), CD19, CD20, GD2 und EGFR.

10. Zusammensetzung nach einem der Ansprüche 1 bis 9, wobei die ABM ein Antikörper oder ein Antigen-bindendes Fragment davon ist.

11. Zusammensetzung nach einem der Ansprüche 1 bis 10, wobei der Anti-LLE-CAR die Sequenz von SEQ ID NO: 11 umfasst, und wobei die extrazelluläre LLE-Bindungsdomäne an eine 6-[6-(Biotinamido)hexanamido]hexanoyl-Einheit bindet.

12. Zusammensetzung nach einem der Ansprüche 1 bis 11, wobei der Anti-LLE-CAR in einer T-Zelle, NK-Zelle, NKT-Zelle, Gamma-Delta-T-Zelle, Treg-Zelle oder Stammzelle exprimiert wird.

## Revendications

1. Composition comprenant
a) un récepteur antigénique chimérique anti-épitope lieur/marqueur (CAR anti-LLE) comprenant
I) un domaine de liaison à l'épitope lieur/marqueur (LLE) extracellulaire,
II) un domaine transmembranaire, et
III) un domaine de transduction du signal,
dans laquelle ledit domaine de liaison au LLE extracellulaire se lie à une molécule de liaison à une cellule cible (TCBM),
b) ladite molécule de liaison à une cellule cible (TCBM) comprenant
i) une fraction de liaison à l'antigène (ABM), dans laquelle ladite ABM se lie spécifiquement à un antigène exprimé sur une cellule cible,
ii) une fraction marqueur (LaM), dans laquelle ladite LaM est une molécule d'origine naturelle chez un sujet ou un dérivé de celle-ci, dans laquelle ladite molécule d'origine naturelle se trouve dans le système circulatoire dudit sujet,
iii) une fraction lieur (LiM) conjuguant ladite ABM et ladite LaM, formant ainsi un épitope lieur/marqueur (LLE), dans laquelle ladite LiM est sélectionnée dans le groupe consistant en des peptides, des chaînes alkyle, des acides alcanoïques, des acides carboxyliques, des farnésyles, des polyéthylène glycols, ou un dérivé de ceux-ci,
dans laquelle ledit domaine de liaison au LLE extracellulaire se lie audit LLE avec une plus grande préférence que ladite molécule d'origine naturelle, dans laquelle ledit domaine de liaison au LLE extracellulaire se lie avec une affinité au moins 10 fois supérieure audit LLE qu'à ladite molécule d'origine naturelle.

2. Composition selon la revendication 1, dans laquelle la valeur k(off) pour la liaison dudit domaine de liaison au LLE extracellulaire à un LLE monomère et à ladite molécule d'origine naturelle est plus élevée que celle pour la liaison à un LLE multimère.

3. Composition selon la revendication 1 ou 2, dans laquelle ledit LLE est généré de manière site spécifique, formant ainsi un épitope comprenant une partie de ladite LaM et une partie de ladite LiM.

4. Composition selon l'une quelconque des revendications 1 à 3, dans laquelle ladite LaM est sélectionnée dans le groupe consistant en des acides aminés, des peptides, la créatinine, la biotine, la biocytine, des hormones, des vitamines, ou un dérivé de ceux-ci.

5. Composition selon l'une quelconque des revendications 1 à 4, dans laquelle ladite LiM est une molécule capable de générer ledit LLE.

6. Composition selon l'une quelconque des revendications 1 à 5, dans laquelle ladite LaM est la biotine ou un dérivé de celle-ci et ladite LiM est une fraction 6-(6-aminohexanamido)hexanoyle ou une fraction 6-aminohexanoyle.

7. Composition selon l'une quelconque des revendications 1 à 6, dans laquelle ledit domaine de liaison au LLE extracellulaire comprend la séquence de SEQ ID NO : 1 et SEQ ID NO : 2.

8. Composition selon l'une quelconque des revendications 1 à 7, dans laquelle ledit antigène qui est lié à ladite ABM est exprimé sur la surface d'une cellule cancéreuse.

9. Composition selon l'une quelconque des revendications 1 à 8, dans laquelle ledit antigène est sélectionné dans le groupe consistant en CD33 (Siglec-3), CD123 (IL3RA), CD135 (FLT-3), CD44 (HCAM), CD44V6, CD47, CD184 (CXCR4), CLEC12A (CLL1), LeY, FRβ, MICA/B, CD305 (LAIR-1), CD366 (TIM-3), CD96 (TACTILE), CD133, CD56, CD29 (ITGB1), CD44 (HCAM), CD47 (IAP), CD66 (CEA), CD112 (Nectin2), CD117 (c-Kit), CD133, CD146 (MCAM), CD155 (PVR), CD171 (L1CAM), CD221 (IGF1), CD227 (MUC1), CD243 (MRD1), CD246 (ALK), CD271 (LNGFR), CD19, CD20, GD2, et EGFR.

10. Composition selon l'une quelconque des revendications 1 à 9, dans laquelle ladite ABM est un anticorps ou un fragment de liaison à l'antigène de celui-ci.

11. Composition selon l'une quelconque des revendications 1 à 10, dans laquelle ledit CAR anti-LLE comprend la séquence de SEQ ID NO : 11, et dans laquelle ledit domaine de liaison au LLE extracellulaire se lie à une fraction 6-[6-(biotinamido)hexanamido] hexanoyle.

12. Composition selon l'une quelconque des revendications 1 à 11, dans laquelle ledit CAR anti-LLE est exprimé dans un lymphocyte T, une cellule NK, une cellule NKT, un lymphocyte T gamma/delta, un lymphocyte Treg, ou une cellule souche.


### SEQUENCE LISTING

<110> Miltenyi Biotec GmbH
<120> Adapter chimeric antigen receptor expressing cells for targeting of multiple antigens
<130> Mil_099
<160> 20
<170> PatentIn version 3.5
<210> 1
   <211> 122
   <212> PRT
   <213> artificial sequence
<220>
   <223> Anti-Biotin antibody (Bio3-18E7.2.A7.A4.A4) heavy chain (VH)
<400> 1
   Glu Val Gln Leu Val Glu Ser Gly Gly Gly Leu Val Lys Pro Gly Gly
   1               5                   10                  15
   Ser Leu Lys Leu Ser Cys Ala Val Ser Gly Phe Thr Phe Ser Asn Tyr
               20                  25                  30
   Ala Met Ser Trp Val Arg Gln Thr Pro Glu Thr Arg Leu Asn Trp Val
           35                  40                  45
   Ala Thr Ile Thr Gly Gly Gly Ser Tyr Thr Tyr Tyr Pro Asp Ser Val
       50                  55                  60
   Lys Gly Arg Phe Thr Ile Ser Arg Asp Asn Ala Lys Asp Thr Leu Tyr
   65                  70                  75                  80
   Leu Gln Met Ser Ser Leu Arg Ser Glu Asp Thr Ala Met Tyr Tyr Cys
                   85                  90                  95
   Val Arg Gln Arg Val Gly Asp Tyr Val Ser Ser Leu Leu Gly Tyr Trp
               100                 105                 110
   Gly Gln Gly Thr Leu Val Thr Val Ser Ala
           115                 120
<210> 2
   <211> 112
   <212> PRT
   <213> artificial sequence
<220>
   <223> Anti-Biotin antibody (Bio3-18E7.2.A7.A4.A4) light chain (VL)
<400> 2
   Asp Val Val Met Thr Gln Thr Pro Leu Ser Leu Pro Val Ser Leu Gly
   1               5                   10                  15
   Asp Gln Ala Ser Ile Ser Cys Arg Ser Ser Gln Ser Leu Val His Ser
               20                  25                  30
   Asn Gly Asn Thr Tyr Leu His Trp Phe Leu Gln Lys Pro Gly Gln Ser
           35                  40                  45
   Pro Asn Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Phe Cys Ser Gln Ser
                   85                  90                  95
   Thr His Val Pro Tyr Thr Phe Gly Gly Gly Thr Lys Leu Glu Ile Lys
               100                 105                 110
<210> 3
   <211> 21
   <212> PRT
   <213> Artificial sequence
<220>
   <223> CD8 Leader sequence (human)
<400> 3
   Met Ala Leu Pro Val Thr Ala Leu Leu Leu Pro Leu Ala Leu Leu Leu
   1               5                   10                  15
   His Ala Ala Arg Pro
               20
<210> 4
   <211> 15
   <212> PRT
   <213> Artificial sequence
<220>
   <223> scFv linker sequence
<400> 4
   Gly Gly Gly Gly Ser Gly Gly Gly Gly Ser Gly Gly Gly Gly Ser
   1               5                   10                  15
<210> 5
   <211> 14
   <212> PRT
   <213> Artificial sequence
<220>
   <223> Spacer #A (human IgG4 HC hinge)
<400> 5
   Ala Ser Glu Ser Lys Tyr Gly Pro Pro Cys Pro Pro Cys Pro
   1               5                   10
<210> 6
   <211> 47
   <212> PRT
   <213> Artificial sequence
<220>
   <223> Spacer #B (human CD8 stalk)
<400> 6
   Ala Ser Thr Thr Thr Pro Ala Pro Arg Pro Pro Thr Pro Ala Pro Thr
   1               5                   10                  15
   Ile Ala Ser Gln Pro Leu Ser Leu Arg Pro Glu Ala Cys Arg Pro Ala
               20                  25                  30
   Ala Gly Gly Ala Val His Thr Arg Gly Leu Asp Phe Ala Cys Asp
           35                  40                  45
<210> 7
   <211> 230
   <212> PRT
   <213> Artificial sequence
<220>
   <223> Spacer #C (human IgG4 HC hinge+CH2+CH3)
<400> 7
   Ala Ser Glu Ser Lys Tyr Gly Pro Pro Cys Pro Pro Cys Pro Ala Pro
   1               5                   10                  15
   Pro Val Ala Gly Pro Ser Val Phe Leu Phe Pro Pro Lys Pro Lys Asp
               20                  25                  30
   Thr Leu Met Ile Ser Arg Thr Pro Glu Val Thr Cys Val Val Val Asp
           35                  40                  45
   Val Ser Gln Glu Asp Pro Glu Val Gln Phe Asn Trp Tyr Val Asp Gly
       50                  55                  60
   Val Glu Val His Asn Ala Lys Thr Lys Pro Arg Glu Glu Gln Phe Gln
   65                  70                  75                  80
   Ser Thr Tyr Arg Val Val Ser Val Leu Thr Val Leu His Gln Asp Trp
                   85                  90                  95
   Leu Asn Gly Lys Glu Tyr Lys Cys Lys Val Ser Asn Lys Gly Leu Pro
               100                 105                 110
   Ser Ser Ile Glu Lys Thr Ile Ser Lys Ala Lys Gly Gln Pro Arg Glu
           115                 120                 125
   Pro Gln Val Tyr Thr Leu Pro Pro Ser Gln Glu Glu Met Thr Lys Asn
       130                 135                 140
   Gln Val Ser Leu Thr Cys Leu Val Lys Gly Phe Tyr Pro Ser Asp Ile
   145                 150                 155                 160
   Ala Val Glu Trp Glu Ser Asn Gly Gln Pro Glu Asn Asn Tyr Lys Thr
                   165                 170                 175
   Thr Pro Pro Val Leu Asp Ser Asp Gly Ser Phe Phe Leu Tyr Ser Arg
               180                 185                 190
   Leu Thr Val Asp Lys Ser Arg Trp Gln Glu Gly Asn Val Phe Ser Cys
           195                 200                 205
   Ser Val Met His Glu Ala Leu His Asn His Tyr Thr Gln Lys Ser Leu
       210                 215                 220
   Ser Leu Ser Leu Gly Lys
   225                 230
<210> 8
   <211> 24
   <212> PRT
   <213> Artificial sequence
<220>
   <223> CD8 Transmembrane (human)
<400> 8
   Ile Tyr Ile Trp Ala Pro Leu Ala Gly Thr Cys Gly Val Leu Leu Leu
   1               5                   10                  15
   Ser Leu Val Ile Thr Leu Tyr Cys
               20
<210> 9
   <211> 42
   <212> PRT
   <213> Artificial sequence
<220>
   <223> 4-1BB (human)
<400> 9
   Lys Arg Gly Arg Lys Lys Leu Leu Tyr Ile Phe Lys Gln Pro Phe Met
   1               5                   10                  15
   Arg Pro Val Gln Thr Thr Gln Glu Glu Asp Gly Cys Ser Cys Arg Phe
               20                  25                  30
   Pro Glu Glu Glu Glu Gly Gly Cys Glu Leu
           35                  40
<210> 10
   <211> 112
   <212> PRT
   <213> Artificial sequence
<220>
   <223> CD3zeta chain (human)
<400> 10
   Arg Val Lys Phe Ser Arg Ser Ala Asp Ala Pro Ala Tyr Gln Gln Gly
   1               5                   10                  15
   Gln Asn Gln Leu Tyr Asn Glu Leu Asn Leu Gly Arg Arg Glu Glu Tyr
               20                  25                  30
   Asp Val Leu Asp Lys Arg Arg Gly Arg Asp Pro Glu Met Gly Gly Lys
           35                  40                  45
   Pro Arg Arg Lys Asn Pro Gln Glu Gly Leu Tyr Asn Glu Leu Gln Lys
       50                  55                  60
   Asp Lys Met Ala Glu Ala Tyr Ser Glu Ile Gly Met Lys Gly Glu Arg
   65                  70                  75                  80
   Arg Arg Gly Lys Gly His Asp Gly Leu Tyr Gln Gly Leu Ser Thr Ala
                   85                  90                  95
   Thr Lys Asp Thr Tyr Asp Ala Leu His Met Gln Ala Leu Pro Pro Arg
               100                 105                 110
<210> 11
   <211> 462
   <212> PRT
   <213> Artificial sequence
<220>
   <223> CD8Leader-anti-BioVL/VH-IgG4Hinge-CD8TM-41BB-CD3z
<400> 11
   Met Ala Leu Pro Val Thr Ala Leu Leu Leu Pro Leu Ala Leu Leu Leu
   1               5                   10                  15
   His Ala Ala Arg Pro Asp Val Val Met Thr Gln Thr Pro Leu Ser Leu
               20                  25                  30
   Pro Val Ser Leu Gly Asp Gln Ala Ser Ile Ser Cys Arg Ser Ser Gln
           35                  40                  45
   Ser Leu Val His Ser Asn Gly Asn Thr Tyr Leu His Trp Phe Leu Gln
       50                  55                  60
   Lys Pro Gly Gln Ser Pro Asn Leu Leu Ile Tyr Lys Val Ser Asn Arg
   65                  70                  75                  80
   Phe Ser Gly Val Pro Asp Arg Phe Ser Gly Ser Gly Ser Gly Thr Asp
                   85                  90                  95
   Phe Thr Leu Lys Ile Ser Arg Val Glu Ala Glu Asp Leu Gly Val Tyr
               100                 105                 110
   Phe Cys Ser Gln Ser Thr His Val Pro Tyr Thr Phe Gly Gly Gly Thr
           115                 120                 125
   Lys Leu Glu Ile Lys Gly Gly Gly Gly Ser Gly Gly Gly Gly Ser Gly
       130                 135                 140
   Gly Gly Gly Ser Glu Val Gln Leu Val Glu Ser Gly Gly Gly Leu Val
   145                 150                 155                 160
   Lys Pro Gly Gly Ser Leu Lys Leu Ser Cys Ala Val Ser Gly Phe Thr
                   165                 170                 175
   Phe Ser Asn Tyr Ala Met Ser Trp Val Arg Gln Thr Pro Glu Thr Arg
               180                 185                 190
   Leu Asn Trp Val Ala Thr Ile Thr Gly Gly Gly Ser Tyr Thr Tyr Tyr
           195                 200                 205
   Pro Asp Ser Val Lys Gly Arg Phe Thr Ile Ser Arg Asp Asn Ala Lys
       210                 215                 220
   Asp Thr Leu Tyr Leu Gln Met Ser Ser Leu Arg Ser Glu Asp Thr Ala
   225                 230                 235                 240
   Met Tyr Tyr Cys Val Arg Gln Arg Val Gly Asp Tyr Val Ser Ser Leu
                   245                 250                 255
   Leu Gly Tyr Trp Gly Gln Gly Thr Leu Val Thr Val Ser Ala Ala Ser
               260                 265                 270
   Glu Ser Lys Tyr Gly Pro Pro Cys Pro Pro Cys Pro Ile Tyr Ile Trp
           275                 280                 285
   Ala Pro Leu Ala Gly Thr Cys Gly Val Leu Leu Leu Ser Leu Val Ile
       290                 295                 300
   Thr Leu Tyr Cys Lys Arg Gly Arg Lys Lys Leu Leu Tyr Ile Phe Lys
   305                 310                 315                 320
   Gln Pro Phe Met Arg Pro Val Gln Thr Thr Gln Glu Glu Asp Gly Cys
                   325                 330                 335
   Ser Cys Arg Phe Pro Glu Glu Glu Glu Gly Gly Cys Glu Leu Arg Val
               340                 345                 350
   Lys Phe Ser Arg Ser Ala Asp Ala Pro Ala Tyr Gln Gln Gly Gln Asn
           355                 360                 365
   Gln Leu Tyr Asn Glu Leu Asn Leu Gly Arg Arg Glu Glu Tyr Asp Val
       370                 375                 380
   Leu Asp Lys Arg Arg Gly Arg Asp Pro Glu Met Gly Gly Lys Pro Arg
   385                 390                 395                 400
   Arg Lys Asn Pro Gln Glu Gly Leu Tyr Asn Glu Leu Gln Lys Asp Lys
                   405                 410                 415
   Met Ala Glu Ala Tyr Ser Glu Ile Gly Met Lys Gly Glu Arg Arg Arg
               420                 425                 430
   Gly Lys Gly His Asp Gly Leu Tyr Gln Gly Leu Ser Thr Ala Thr Lys
           435                 440                 445
   Asp Thr Tyr Asp Ala Leu His Met Gln Ala Leu Pro Pro Arg
       450                 455                 460
<210> 12
   <211> 462
   <212> PRT
   <213> Artificial sequence
<220>
   <223> CD8Leader-anti-BioVH/VL-IgG4Hinge-CD8TM-41BB-CD3z
<400> 12
   Met Ala Leu Pro Val Thr Ala Leu Leu Leu Pro Leu Ala Leu Leu Leu
   1               5                   10                  15
   His Ala Ala Arg Pro Glu Val Gln Leu Val Glu Ser Gly Gly Gly Leu
               20                  25                  30
   Val Lys Pro Gly Gly Ser Leu Lys Leu Ser Cys Ala Val Ser Gly Phe
           35                  40                  45
   Thr Phe Ser Asn Tyr Ala Met Ser Trp Val Arg Gln Thr Pro Glu Thr
       50                  55                  60
   Arg Leu Asn Trp Val Ala Thr Ile Thr Gly Gly Gly Ser Tyr Thr Tyr
   65                  70                  75                  80
   Tyr Pro Asp Ser Val Lys Gly Arg Phe Thr Ile Ser Arg Asp Asn Ala
                   85                  90                  95
   Lys Asp Thr Leu Tyr Leu Gln Met Ser Ser Leu Arg Ser Glu Asp Thr
               100                 105                 110
   Ala Met Tyr Tyr Cys Val Arg Gln Arg Val Gly Asp Tyr Val Ser Ser
           115                 120                 125
   Leu Leu Gly Tyr Trp Gly Gln Gly Thr Leu Val Thr Val Ser Ala Gly
       130                 135                 140
   Gly Gly Gly Ser Gly Gly Gly Gly Ser Gly Gly Gly Gly Ser Asp Val
   145                 150                 155                 160
   Val Met Thr Gln Thr Pro Leu Ser Leu Pro Val Ser Leu Gly Asp Gln
                   165                 170                 175
   Ala Ser Ile Ser Cys Arg Ser Ser Gln Ser Leu Val His Ser Asn Gly
               180                 185                 190
   Asn Thr Tyr Leu His Trp Phe Leu Gln Lys Pro Gly Gln Ser Pro Asn
           195                 200                 205
   Leu Leu Ile Tyr Lys Val Ser Asn Arg Phe Ser Gly Val Pro Asp Arg
       210                 215                 220
   Phe Ser Gly Ser Gly Ser Gly Thr Asp Phe Thr Leu Lys Ile Ser Arg
   225                 230                 235                 240
   Val Glu Ala Glu Asp Leu Gly Val Tyr Phe Cys Ser Gln Ser Thr His
                   245                 250                 255
   Val Pro Tyr Thr Phe Gly Gly Gly Thr Lys Leu Glu Ile Lys Ala Ser
               260                 265                 270
   Glu Ser Lys Tyr Gly Pro Pro Cys Pro Pro Cys Pro Ile Tyr Ile Trp
           275                 280                 285
   Ala Pro Leu Ala Gly Thr Cys Gly Val Leu Leu Leu Ser Leu Val Ile
       290                 295                 300
   Thr Leu Tyr Cys Lys Arg Gly Arg Lys Lys Leu Leu Tyr Ile Phe Lys
   305                 310                 315                 320
   Gln Pro Phe Met Arg Pro Val Gln Thr Thr Gln Glu Glu Asp Gly Cys
                   325                 330                 335
   Ser Cys Arg Phe Pro Glu Glu Glu Glu Gly Gly Cys Glu Leu Arg Val
               340                 345                 350
   Lys Phe Ser Arg Ser Ala Asp Ala Pro Ala Tyr Gln Gln Gly Gln Asn
           355                 360                 365
   Gln Leu Tyr Asn Glu Leu Asn Leu Gly Arg Arg Glu Glu Tyr Asp Val
       370                 375                 380
   Leu Asp Lys Arg Arg Gly Arg Asp Pro Glu Met Gly Gly Lys Pro Arg
   385                 390                 395                 400
   Arg Lys Asn Pro Gln Glu Gly Leu Tyr Asn Glu Leu Gln Lys Asp Lys
                   405                 410                 415
   Met Ala Glu Ala Tyr Ser Glu Ile Gly Met Lys Gly Glu Arg Arg Arg
               420                 425                 430
   Gly Lys Gly His Asp Gly Leu Tyr Gln Gly Leu Ser Thr Ala Thr Lys
           435                 440                 445
   Asp Thr Tyr Asp Ala Leu His Met Gln Ala Leu Pro Pro Arg
       450                 455                 460
<210> 13
   <211> 495
   <212> PRT
   <213> Artificial sequence
<220>
   <223> CD8Leader-anti-BioVL/VH-CD8Stalk-CD8TM-41BB-CD3z
<400> 13
   Met Ala Leu Pro Val Thr Ala Leu Leu Leu Pro Leu Ala Leu Leu Leu
   1               5                   10                  15
   His Ala Ala Arg Pro Asp Val Val Met Thr Gln Thr Pro Leu Ser Leu
               20                  25                  30
   Pro Val Ser Leu Gly Asp Gln Ala Ser Ile Ser Cys Arg Ser Ser Gln
           35                  40                  45
   Ser Leu Val His Ser Asn Gly Asn Thr Tyr Leu His Trp Phe Leu Gln
       50                  55                  60
   Lys Pro Gly Gln Ser Pro Asn Leu Leu Ile Tyr Lys Val Ser Asn Arg
   65                  70                  75                  80
   Phe Ser Gly Val Pro Asp Arg Phe Ser Gly Ser Gly Ser Gly Thr Asp
                   85                  90                  95
   Phe Thr Leu Lys Ile Ser Arg Val Glu Ala Glu Asp Leu Gly Val Tyr
               100                 105                 110
   Phe Cys Ser Gln Ser Thr His Val Pro Tyr Thr Phe Gly Gly Gly Thr
           115                 120                 125
   Lys Leu Glu Ile Lys Gly Gly Gly Gly Ser Gly Gly Gly Gly Ser Gly
       130                 135                 140
   Gly Gly Gly Ser Glu Val Gln Leu Val Glu Ser Gly Gly Gly Leu Val
   145                 150                 155                 160
   Lys Pro Gly Gly Ser Leu Lys Leu Ser Cys Ala Val Ser Gly Phe Thr
                   165                 170                 175
   Phe Ser Asn Tyr Ala Met Ser Trp Val Arg Gln Thr Pro Glu Thr Arg
               180                 185                 190
   Leu Asn Trp Val Ala Thr Ile Thr Gly Gly Gly Ser Tyr Thr Tyr Tyr
           195                 200                 205
   Pro Asp Ser Val Lys Gly Arg Phe Thr Ile Ser Arg Asp Asn Ala Lys
       210                 215                 220
   Asp Thr Leu Tyr Leu Gln Met Ser Ser Leu Arg Ser Glu Asp Thr Ala
   225                 230                 235                 240
   Met Tyr Tyr Cys Val Arg Gln Arg Val Gly Asp Tyr Val Ser Ser Leu
                   245                 250                 255
   Leu Gly Tyr Trp Gly Gln Gly Thr Leu Val Thr Val Ser Ala Ala Ser
               260                 265                 270
   Thr Thr Thr Pro Ala Pro Arg Pro Pro Thr Pro Ala Pro Thr Ile Ala
           275                 280                 285
   Ser Gln Pro Leu Ser Leu Arg Pro Glu Ala Cys Arg Pro Ala Ala Gly
       290                 295                 300
   Gly Ala Val His Thr Arg Gly Leu Asp Phe Ala Cys Asp Ile Tyr Ile
   305                 310                 315                 320
   Trp Ala Pro Leu Ala Gly Thr Cys Gly Val Leu Leu Leu Ser Leu Val
                   325                 330                 335
   Ile Thr Leu Tyr Cys Lys Arg Gly Arg Lys Lys Leu Leu Tyr Ile Phe
               340                 345                 350
   Lys Gln Pro Phe Met Arg Pro Val Gln Thr Thr Gln Glu Glu Asp Gly
           355                 360                 365
   Cys Ser Cys Arg Phe Pro Glu Glu Glu Glu Gly Gly Cys Glu Leu Arg
       370                 375                 380
   Val Lys Phe Ser Arg Ser Ala Asp Ala Pro Ala Tyr Gln Gln Gly Gln
   385                 390                 395                 400
   Asn Gln Leu Tyr Asn Glu Leu Asn Leu Gly Arg Arg Glu Glu Tyr Asp
                   405                 410                 415
   Val Leu Asp Lys Arg Arg Gly Arg Asp Pro Glu Met Gly Gly Lys Pro
               420                 425                 430
   Arg Arg Lys Asn Pro Gln Glu Gly Leu Tyr Asn Glu Leu Gln Lys Asp
           435                 440                 445
   Lys Met Ala Glu Ala Tyr Ser Glu Ile Gly Met Lys Gly Glu Arg Arg
       450                 455                 460
   Arg Gly Lys Gly His Asp Gly Leu Tyr Gln Gly Leu Ser Thr Ala Thr
   465                 470                 475                 480
   Lys Asp Thr Tyr Asp Ala Leu His Met Gln Ala Leu Pro Pro Arg
                   485                 490                 495
<210> 14
   <211> 495
   <212> PRT
   <213> Artificial sequence
<220>
   <223> CD8Leader-anti-BioVH/VL-CD8Stalk-CD8TM-41BB-CD3z
<400> 14
   Met Ala Leu Pro Val Thr Ala Leu Leu Leu Pro Leu Ala Leu Leu Leu
   1               5                   10                  15
   His Ala Ala Arg Pro Glu Val Gln Leu Val Glu Ser Gly Gly Gly Leu
               20                  25                  30
   Val Lys Pro Gly Gly Ser Leu Lys Leu Ser Cys Ala Val Ser Gly Phe
           35                  40                  45
   Thr Phe Ser Asn Tyr Ala Met Ser Trp Val Arg Gln Thr Pro Glu Thr
       50                  55                  60
   Arg Leu Asn Trp Val Ala Thr Ile Thr Gly Gly Gly Ser Tyr Thr Tyr
   65                  70                  75                  80
   Tyr Pro Asp Ser Val Lys Gly Arg Phe Thr Ile Ser Arg Asp Asn Ala
                   85                  90                  95
   Lys Asp Thr Leu Tyr Leu Gln Met Ser Ser Leu Arg Ser Glu Asp Thr
               100                 105                 110
   Ala Met Tyr Tyr Cys Val Arg Gln Arg Val Gly Asp Tyr Val Ser Ser
           115                 120                 125
   Leu Leu Gly Tyr Trp Gly Gln Gly Thr Leu Val Thr Val Ser Ala Gly
       130                 135                 140
   Gly Gly Gly Ser Gly Gly Gly Gly Ser Gly Gly Gly Gly Ser Asp Val
   145                 150                 155                 160
   Val Met Thr Gln Thr Pro Leu Ser Leu Pro Val Ser Leu Gly Asp Gln
                   165                 170                 175
   Ala Ser Ile Ser Cys Arg Ser Ser Gln Ser Leu Val His Ser Asn Gly
               180                 185                 190
   Asn Thr Tyr Leu His Trp Phe Leu Gln Lys Pro Gly Gln Ser Pro Asn
           195                 200                 205
   Leu Leu Ile Tyr Lys Val Ser Asn Arg Phe Ser Gly Val Pro Asp Arg
       210                 215                 220
   Phe Ser Gly Ser Gly Ser Gly Thr Asp Phe Thr Leu Lys Ile Ser Arg
   225                 230                 235                 240
   Val Glu Ala Glu Asp Leu Gly Val Tyr Phe Cys Ser Gln Ser Thr His
                   245                 250                 255
   Val Pro Tyr Thr Phe Gly Gly Gly Thr Lys Leu Glu Ile Lys Ala Ser
               260                 265                 270
   Thr Thr Thr Pro Ala Pro Arg Pro Pro Thr Pro Ala Pro Thr Ile Ala
           275                 280                 285
   Ser Gln Pro Leu Ser Leu Arg Pro Glu Ala Cys Arg Pro Ala Ala Gly
       290                 295                 300
   Gly Ala Val His Thr Arg Gly Leu Asp Phe Ala Cys Asp Ile Tyr Ile
   305                 310                 315                 320
   Trp Ala Pro Leu Ala Gly Thr Cys Gly Val Leu Leu Leu Ser Leu Val
                   325                 330                 335
   Ile Thr Leu Tyr Cys Lys Arg Gly Arg Lys Lys Leu Leu Tyr Ile Phe
               340                 345                 350
   Lys Gln Pro Phe Met Arg Pro Val Gln Thr Thr Gln Glu Glu Asp Gly
           355                 360                 365
   Cys Ser Cys Arg Phe Pro Glu Glu Glu Glu Gly Gly Cys Glu Leu Arg
       370                 375                 380
   Val Lys Phe Ser Arg Ser Ala Asp Ala Pro Ala Tyr Gln Gln Gly Gln
   385                 390                 395                 400
   Asn Gln Leu Tyr Asn Glu Leu Asn Leu Gly Arg Arg Glu Glu Tyr Asp
                   405                 410                 415
   Val Leu Asp Lys Arg Arg Gly Arg Asp Pro Glu Met Gly Gly Lys Pro
               420                 425                 430
   Arg Arg Lys Asn Pro Gln Glu Gly Leu Tyr Asn Glu Leu Gln Lys Asp
           435                 440                 445
   Lys Met Ala Glu Ala Tyr Ser Glu Ile Gly Met Lys Gly Glu Arg Arg
       450                 455                 460
   Arg Gly Lys Gly His Asp Gly Leu Tyr Gln Gly Leu Ser Thr Ala Thr
   465 470 475 480
   Lys Asp Thr Tyr Asp Ala Leu His Met Gln Ala Leu Pro Pro Arg
   485 490 495
<210> 15
   <211> 678
   <212> PRT
   <213> Artificial sequence
<220>
   <223> CD8Leader-anti-BioVL/VH-IgG4Hinge+CH2+CH3-CD8TM-41BB-CD3z
<400> 15
   Met Ala Leu Pro Val Thr Ala Leu Leu Leu Pro Leu Ala Leu Leu Leu
   1 5 10 15
   His Ala Ala Arg Pro Asp Val Val Met Thr Gln Thr Pro Leu Ser Leu
   20 25 30
   Pro Val Ser Leu Gly Asp Gln Ala Ser Ile Ser Cys Arg Ser Ser Gln
   35 40 45
   Ser Leu Val His Ser Asn Gly Asn Thr Tyr Leu His Trp Phe Leu Gln
   50 55 60
   Lys Pro Gly Gln Ser Pro Asn Leu Leu Ile Tyr Lys Val Ser Asn Arg
   65 70 75 80
   Phe Ser Gly Val Pro Asp Arg Phe Ser Gly Ser Gly Ser Gly Thr Asp
   85 90 95
   Phe Thr Leu Lys Ile Ser Arg Val Glu Ala Glu Asp Leu Gly Val Tyr
   100 105 110
   Phe Cys Ser Gln Ser Thr His Val Pro Tyr Thr Phe Gly Gly Gly Thr
   115 120 125
   Lys Leu Glu Ile Lys Gly Gly Gly Gly Ser Gly Gly Gly Gly Ser Gly
   130 135 140
   Gly Gly Gly Ser Glu Val Gln Leu Val Glu Ser Gly Gly Gly Leu Val
   145 150 155 160
   Lys Pro Gly Gly Ser Leu Lys Leu Ser Cys Ala Val Ser Gly Phe Thr
   165 170 175
   Phe Ser Asn Tyr Ala Met Ser Trp Val Arg Gln Thr Pro Glu Thr Arg
               180                 185                 190
   Leu Asn Trp Val Ala Thr Ile Thr Gly Gly Gly Ser Tyr Thr Tyr Tyr
           195                 200                 205
   Pro Asp Ser Val Lys Gly Arg Phe Thr Ile Ser Arg Asp Asn Ala Lys
       210                 215                 220
   Asp Thr Leu Tyr Leu Gln Met Ser Ser Leu Arg Ser Glu Asp Thr Ala
   225                 230                 235                 240
   Met Tyr Tyr Cys Val Arg Gln Arg Val Gly Asp Tyr Val Ser Ser Leu
                   245                 250                 255
   Leu Gly Tyr Trp Gly Gln Gly Thr Leu Val Thr Val Ser Ala Ala Ser
               260                 265                 270
   Glu Ser Lys Tyr Gly Pro Pro Cys Pro Pro Cys Pro Ala Pro Pro Val
           275                 280                 285
   Ala Gly Pro Ser Val Phe Leu Phe Pro Pro Lys Pro Lys Asp Thr Leu
       290                 295                 300
   Met Ile Ser Arg Thr Pro Glu Val Thr Cys Val Val Val Asp Val Ser
   305                 310                 315                 320
   Gln Glu Asp Pro Glu Val Gln Phe Asn Trp Tyr Val Asp Gly Val Glu
                   325                 330                 335
   Val His Asn Ala Lys Thr Lys Pro Arg Glu Glu Gln Phe Gln Ser Thr
               340                 345                 350
   Tyr Arg Val Val Ser Val Leu Thr Val Leu His Gln Asp Trp Leu Asn
           355                 360                 365
   Gly Lys Glu Tyr Lys Cys Lys Val Ser Asn Lys Gly Leu Pro Ser Ser
       370                 375                 380
   Ile Glu Lys Thr Ile Ser Lys Ala Lys Gly Gln Pro Arg Glu Pro Gln
   385                 390                 395                 400
   Val Tyr Thr Leu Pro Pro Ser Gln Glu Glu Met Thr Lys Asn Gln Val
                   405                 410                 415
   Ser Leu Thr Cys Leu Val Lys Gly Phe Tyr Pro Ser Asp Ile Ala Val
               420                 425                 430
   Glu Trp Glu Ser Asn Gly Gln Pro Glu Asn Asn Tyr Lys Thr Thr Pro
           435                 440                 445
   Pro Val Leu Asp Ser Asp Gly Ser Phe Phe Leu Tyr Ser Arg Leu Thr
       450                 455                 460
   Val Asp Lys Ser Arg Trp Gln Glu Gly Asn Val Phe Ser Cys Ser Val
   465                 470                 475                 480
   Met His Glu Ala Leu His Asn His Tyr Thr Gln Lys Ser Leu Ser Leu
                   485                 490                 495
   Ser Leu Gly Lys Ile Tyr Ile Trp Ala Pro Leu Ala Gly Thr Cys Gly
               500                 505                 510
   Val Leu Leu Leu Ser Leu Val Ile Thr Leu Tyr Cys Lys Arg Gly Arg
           515                 520                 525
   Lys Lys Leu Leu Tyr Ile Phe Lys Gln Pro Phe Met Arg Pro Val Gln
       530                 535                 540
   Thr Thr Gln Glu Glu Asp Gly Cys Ser Cys Arg Phe Pro Glu Glu Glu
   545                 550                 555                 560
   Glu Gly Gly Cys Glu Leu Arg Val Lys Phe Ser Arg Ser Ala Asp Ala
                   565                 570                 575
   Pro Ala Tyr Gln Gln Gly Gln Asn Gln Leu Tyr Asn Glu Leu Asn Leu
               580                 585                 590
   Gly Arg Arg Glu Glu Tyr Asp Val Leu Asp Lys Arg Arg Gly Arg Asp
           595                 600                 605
   Pro Glu Met Gly Gly Lys Pro Arg Arg Lys Asn Pro Gln Glu Gly Leu
       610                 615                 620
   Tyr Asn Glu Leu Gln Lys Asp Lys Met Ala Glu Ala Tyr Ser Glu Ile
   625                 630                 635                 640
   Gly Met Lys Gly Glu Arg Arg Arg Gly Lys Gly His Asp Gly Leu Tyr
                   645                 650                 655
   Gln Gly Leu Ser Thr Ala Thr Lys Asp Thr Tyr Asp Ala Leu His Met
               660                 665                 670
   Gln Ala Leu Pro Pro Arg
   675
<210> 16
   <211> 678
   <212> PRT
   <213> Artificial sequence
<220>
   <223> CD8Leader-anti-BioVH/VL- IgG4Hinge+CH2+CH3-CD8TM-41BB-CD3z
<400> 16
   Met Ala Leu Pro Val Thr Ala Leu Leu Leu Pro Leu Ala Leu Leu Leu
   1               5                   10                  15
   His Ala Ala Arg Pro Glu Val Gln Leu Val Glu Ser Gly Gly Gly Leu
               20                  25                  30
   Val Lys Pro Gly Gly Ser Leu Lys Leu Ser Cys Ala Val Ser Gly Phe
           35                  40                  45
   Thr Phe Ser Asn Tyr Ala Met Ser Trp Val Arg Gln Thr Pro Glu Thr
       50                  55                  60
   Arg Leu Asn Trp Val Ala Thr Ile Thr Gly Gly Gly Ser Tyr Thr Tyr
   65                  70                  75                  80
   Tyr Pro Asp Ser Val Lys Gly Arg Phe Thr Ile Ser Arg Asp Asn Ala
                   85                  90                  95
   Lys Asp Thr Leu Tyr Leu Gln Met Ser Ser Leu Arg Ser Glu Asp Thr
               100                 105                 110
   Ala Met Tyr Tyr Cys Val Arg Gln Arg Val Gly Asp Tyr Val Ser Ser
           115                 120                 125
   Leu Leu Gly Tyr Trp Gly Gln Gly Thr Leu Val Thr Val Ser Ala Gly
       130                 135                 140
   Gly Gly Gly Ser Gly Gly Gly Gly Ser Gly Gly Gly Gly Ser Asp Val
   145                 150                 155                 160
   Val Met Thr Gln Thr Pro Leu Ser Leu Pro Val Ser Leu Gly Asp Gln
                   165                 170                 175
   Ala Ser Ile Ser Cys Arg Ser Ser Gln Ser Leu Val His Ser Asn Gly
               180                 185                 190
   Asn Thr Tyr Leu His Trp Phe Leu Gln Lys Pro Gly Gln Ser Pro Asn
           195                 200                 205
   Leu Leu Ile Tyr Lys Val Ser Asn Arg Phe Ser Gly Val Pro Asp Arg
       210                 215                 220
   Phe Ser Gly Ser Gly Ser Gly Thr Asp Phe Thr Leu Lys Ile Ser Arg
   225                 230                 235                 240
   Val Glu Ala Glu Asp Leu Gly Val Tyr Phe Cys Ser Gln Ser Thr His
                   245                 250                 255
   Val Pro Tyr Thr Phe Gly Gly Gly Thr Lys Leu Glu Ile Lys Ala Ser
               260                 265                 270
   Glu Ser Lys Tyr Gly Pro Pro Cys Pro Pro Cys Pro Ala Pro Pro Val
           275                 280                 285
   Ala Gly Pro Ser Val Phe Leu Phe Pro Pro Lys Pro Lys Asp Thr Leu
       290                 295                 300
   Met Ile Ser Arg Thr Pro Glu Val Thr Cys Val Val Val Asp Val Ser
   305                 310                 315                 320
   Gln Glu Asp Pro Glu Val Gln Phe Asn Trp Tyr Val Asp Gly Val Glu
                   325                 330                 335
   Val His Asn Ala Lys Thr Lys Pro Arg Glu Glu Gln Phe Gln Ser Thr
               340                 345                 350
   Tyr Arg Val Val Ser Val Leu Thr Val Leu His Gln Asp Trp Leu Asn
           355                 360                 365
   Gly Lys Glu Tyr Lys Cys Lys Val Ser Asn Lys Gly Leu Pro Ser Ser
       370                 375                 380
   Ile Glu Lys Thr Ile Ser Lys Ala Lys Gly Gln Pro Arg Glu Pro Gln
   385                 390                 395                 400
   Val Tyr Thr Leu Pro Pro Ser Gln Glu Glu Met Thr Lys Asn Gln Val
                   405                 410                 415
   Ser Leu Thr Cys Leu Val Lys Gly Phe Tyr Pro Ser Asp Ile Ala Val
               420                 425                 430
   Glu Trp Glu Ser Asn Gly Gln Pro Glu Asn Asn Tyr Lys Thr Thr Pro
           435                 440                 445
   Pro Val Leu Asp Ser Asp Gly Ser Phe Phe Leu Tyr Ser Arg Leu Thr
   450 455 460
   Val Asp Lys Ser Arg Trp Gln Glu Gly Asn Val Phe Ser Cys Ser Val
   465 470 475 480
   Met His Glu Ala Leu His Asn His Tyr Thr Gln Lys Ser Leu Ser Leu
   485 490 495
   Ser Leu Gly Lys Ile Tyr Ile Trp Ala Pro Leu Ala Gly Thr Cys Gly
   500 505 510
   Val Leu Leu Leu Ser Leu Val Ile Thr Leu Tyr Cys Lys Arg Gly Arg
   515 520 525
   Lys Lys Leu Leu Tyr Ile Phe Lys Gln Pro Phe Met Arg Pro Val Gln
   530 535 540
   Thr Thr Gln Glu Glu Asp Gly Cys Ser Cys Arg Phe Pro Glu Glu Glu
   545 550 555 560
   Glu Gly Gly Cys Glu Leu Arg Val Lys Phe Ser Arg Ser Ala Asp Ala
   565 570 575
   Pro Ala Tyr Gln Gln Gly Gln Asn Gln Leu Tyr Asn Glu Leu Asn Leu
   580 585 590
   Gly Arg Arg Glu Glu Tyr Asp Val Leu Asp Lys Arg Arg Gly Arg Asp
   595 600 605
   Pro Glu Met Gly Gly Lys Pro Arg Arg Lys Asn Pro Gln Glu Gly Leu
   610 615 620
   Tyr Asn Glu Leu Gln Lys Asp Lys Met Ala Glu Ala Tyr Ser Glu Ile
   625 630 635 640
   Gly Met Lys Gly Glu Arg Arg Arg Gly Lys Gly His Asp Gly Leu Tyr
   645 650 655
   Gln Gly Leu Ser Thr Ala Thr Lys Asp Thr Tyr Asp Ala Leu His Met
   660 665 670
   Gln Ala Leu Pro Pro Arg
   675
<210> 17
   <211> 774
   <212> PRT
   <213> Artificial sequence
<220>
   <223> CD8Leader-anti-BioVL/VH-CH1+IgG4Hinge+CH2+CH3-CD8TM-41BB-CD3z
<400> 17
   Met Ala Leu Pro Val Thr Ala Leu Leu Leu Pro Leu Ala Leu Leu Leu
   1               5                   10                  15
   His Ala Ala Arg Pro Asp Val Val Met Thr Gln Thr Pro Leu Ser Leu
               20                  25                  30
   Pro Val Ser Leu Gly Asp Gln Ala Ser Ile Ser Cys Arg Ser Ser Gln
           35                  40                  45
   Ser Leu Val His Ser Asn Gly Asn Thr Tyr Leu His Trp Phe Leu Gln
       50                  55                  60
   Lys Pro Gly Gln Ser Pro Asn Leu Leu Ile Tyr Lys Val Ser Asn Arg
   65                  70                  75                  80
   Phe Ser Gly Val Pro Asp Arg Phe Ser Gly Ser Gly Ser Gly Thr Asp
                   85                  90                  95
   Phe Thr Leu Lys Ile Ser Arg Val Glu Ala Glu Asp Leu Gly Val Tyr
               100                 105                 110
   Phe Cys Ser Gln Ser Thr His Val Pro Tyr Thr Phe Gly Gly Gly Thr
           115                 120                 125
   Lys Leu Glu Ile Lys Gly Gly Gly Gly Ser Gly Gly Gly Gly Ser Gly
       130                 135                 140
   Gly Gly Gly Ser Glu Val Gln Leu Val Glu Ser Gly Gly Gly Leu Val
   145                 150                 155                 160
   Lys Pro Gly Gly Ser Leu Lys Leu Ser Cys Ala Val Ser Gly Phe Thr
                   165                 170                 175
   Phe Ser Asn Tyr Ala Met Ser Trp Val Arg Gln Thr Pro Glu Thr Arg
               180                 185                 190
   Leu Asn Trp Val Ala Thr Ile Thr Gly Gly Gly Ser Tyr Thr Tyr Tyr
           195                 200                 205
   Pro Asp Ser Val Lys Gly Arg Phe Thr Ile Ser Arg Asp Asn Ala Lys
       210                 215                 220
   Asp Thr Leu Tyr Leu Gln Met Ser Ser Leu Arg Ser Glu Asp Thr Ala
   225 230 235 240
   Met Tyr Tyr Cys Val Arg Gln Arg Val Gly Asp Tyr Val Ser Ser Leu
   245 250 255
   Leu Gly Tyr Trp Gly Gln Gly Thr Leu Val Thr Val Ser Ala Ala Ser
   260 265 270
   Thr Lys Gly Pro Ser Val Phe Pro Leu Ala Pro Ser Ser Arg Ser Thr
   275 280 285
   Ser Glu Ser Thr Ala Ala Leu Gly Cys Leu Val Lys Asp Tyr Phe Pro
   290 295 300
   Glu Pro Val Thr Val Ser Trp Asn Ser Gly Ala Leu Thr Ser Gly Val
   305 310 315 320
   His Thr Phe Pro Ala Val Leu Gln Ser Ser Gly Leu Tyr Ser Leu Ser
   325 330 335
   Ser Val Val Thr Val Pro Ser Ser Ser Leu Gly Thr Lys Thr Tyr Thr
   340 345 350
   Cys Asn Val Asp His Lys Pro Ser Asn Thr Lys Val Asp Lys Arg Val
   355 360 365
   Glu Ser Lys Tyr Gly Pro Pro Cys Pro Pro Cys Pro Ala Pro Pro Val
   370 375 380
   Ala Gly Pro Ser Val Phe Leu Phe Pro Pro Lys Pro Lys Asp Thr Leu
   385 390 395 400
   Met Ile Ser Arg Thr Pro Glu Val Thr Cys Val Val Val Asp Val Ser
   405 410 415
   Gln Glu Asp Pro Glu Val Gln Phe Asn Trp Tyr Val Asp Gly Val Glu
   420 425 430
   Val His Asn Ala Lys Thr Lys Pro Arg Glu Glu Gln Phe Gln Ser Thr
   435 440 445
   Tyr Arg Val Val Ser Val Leu Thr Val Leu His Gln Asp Trp Leu Asn
   450 455 460
   Gly Lys Glu Tyr Lys Cys Lys Val Ser Asn Lys Gly Leu Pro Ser Ser
   465 470 475 480
   Ile Glu Lys Thr Ile Ser Lys Ala Lys Gly Gln Pro Arg Glu Pro Gln
                   485                 490                 495
   Val Tyr Thr Leu Pro Pro Ser Gln Glu Glu Met Thr Lys Asn Gln Val
               500                 505                 510
   Ser Leu Thr Cys Leu Val Lys Gly Phe Tyr Pro Ser Asp Ile Ala Val
           515                 520                 525
   Glu Trp Glu Ser Asn Gly Gln Pro Glu Asn Asn Tyr Lys Thr Thr Pro
       530                 535                 540
   Pro Val Leu Asp Ser Asp Gly Ser Phe Phe Leu Tyr Ser Arg Leu Thr
   545                 550                 555                 560
   Val Asp Lys Ser Arg Trp Gln Glu Gly Asn Val Phe Ser Cys Ser Val
                   565                 570                 575
   Met His Glu Ala Leu His Asn His Tyr Thr Gln Lys Ser Leu Ser Leu
               580                 585                 590
   Ser Leu Gly Lys Ile Tyr Ile Trp Ala Pro Leu Ala Gly Thr Cys Gly
           595                 600                 605
   Val Leu Leu Leu Ser Leu Val Ile Thr Leu Tyr Cys Lys Arg Gly Arg
       610                 615                 620
   Lys Lys Leu Leu Tyr Ile Phe Lys Gln Pro Phe Met Arg Pro Val Gln
   625                 630                 635                 640
   Thr Thr Gln Glu Glu Asp Gly Cys Ser Cys Arg Phe Pro Glu Glu Glu
                   645                 650                 655
   Glu Gly Gly Cys Glu Leu Arg Val Lys Phe Ser Arg Ser Ala Asp Ala
               660                 665                 670
   Pro Ala Tyr Gln Gln Gly Gln Asn Gln Leu Tyr Asn Glu Leu Asn Leu
           675                 680                 685
   Gly Arg Arg Glu Glu Tyr Asp Val Leu Asp Lys Arg Arg Gly Arg Asp
       690                 695                 700
   Pro Glu Met Gly Gly Lys Pro Arg Arg Lys Asn Pro Gln Glu Gly Leu
   705                 710                 715                 720
   Tyr Asn Glu Leu Gln Lys Asp Lys Met Ala Glu Ala Tyr Ser Glu Ile
                   725                 730                 735
   Gly Met Lys Gly Glu Arg Arg Arg Gly Lys Gly His Asp Gly Leu Tyr
   740 745 750
   Gln Gly Leu Ser Thr Ala Thr Lys Asp Thr Tyr Asp Ala Leu His Met
   755 760 765
   Gln Ala Leu Pro Pro Arg
   770
<210> 18
   <211> 774
   <212> PRT
   <213> Artificial sequence
<220>
   <223> CD8Leader-anti-BioVH/VL-CH1+IgG4Hinge+CH2+CH3-CD8TM-41BB-CD3z
<400> 18
   Met Ala Leu Pro Val Thr Ala Leu Leu Leu Pro Leu Ala Leu Leu Leu
   1 5 10 15
   His Ala Ala Arg Pro Glu Val Gln Leu Val Glu Ser Gly Gly Gly Leu
   20 25 30
   Val Lys Pro Gly Gly Ser Leu Lys Leu Ser Cys Ala Val Ser Gly Phe
   35 40 45
   Thr Phe Ser Asn Tyr Ala Met Ser Trp Val Arg Gln Thr Pro Glu Thr
   50 55 60
   Arg Leu Asn Trp Val Ala Thr Ile Thr Gly Gly Gly Ser Tyr Thr Tyr
   65 70 75 80
   Tyr Pro Asp Ser Val Lys Gly Arg Phe Thr Ile Ser Arg Asp Asn Ala
   85 90 95
   Lys Asp Thr Leu Tyr Leu Gln Met Ser Ser Leu Arg Ser Glu Asp Thr
   100 105 110
   Ala Met Tyr Tyr Cys Val Arg Gln Arg Val Gly Asp Tyr Val Ser Ser
   115 120 125
   Leu Leu Gly Tyr Trp Gly Gln Gly Thr Leu Val Thr Val Ser Ala Gly
   130 135 140
   Gly Gly Gly Ser Gly Gly Gly Gly Ser Gly Gly Gly Gly Ser Asp Val
   145 150 155 160
   Val Met Thr Gln Thr Pro Leu Ser Leu Pro Val Ser Leu Gly Asp Gln
                   165                 170                 175
   Ala Ser Ile Ser Cys Arg Ser Ser Gln Ser Leu Val His Ser Asn Gly
               180                 185                 190
   Asn Thr Tyr Leu His Trp Phe Leu Gln Lys Pro Gly Gln Ser Pro Asn
           195                 200                 205
   Leu Leu Ile Tyr Lys Val Ser Asn Arg Phe Ser Gly Val Pro Asp Arg
       210                 215                 220
   Phe Ser Gly Ser Gly Ser Gly Thr Asp Phe Thr Leu Lys Ile Ser Arg
   225                 230                 235                 240
   Val Glu Ala Glu Asp Leu Gly Val Tyr Phe Cys Ser Gln Ser Thr His
                   245                 250                 255
   Val Pro Tyr Thr Phe Gly Gly Gly Thr Lys Leu Glu Ile Lys Ala Ser
               260                 265                 270
   Thr Lys Gly Pro Ser Val Phe Pro Leu Ala Pro Ser Ser Arg Ser Thr
           275                 280                 285
   Ser Glu Ser Thr Ala Ala Leu Gly Cys Leu Val Lys Asp Tyr Phe Pro
       290                 295                 300
   Glu Pro Val Thr Val Ser Trp Asn Ser Gly Ala Leu Thr Ser Gly Val
   305                 310                 315                 320
   His Thr Phe Pro Ala Val Leu Gln Ser Ser Gly Leu Tyr Ser Leu Ser
                   325                 330                 335
   Ser Val Val Thr Val Pro Ser Ser Ser Leu Gly Thr Lys Thr Tyr Thr
               340                 345                 350
   Cys Asn Val Asp His Lys Pro Ser Asn Thr Lys Val Asp Lys Arg Val
           355                 360                 365
   Glu Ser Lys Tyr Gly Pro Pro Cys Pro Pro Cys Pro Ala Pro Pro Val
       370                 375                 380
   Ala Gly Pro Ser Val Phe Leu Phe Pro Pro Lys Pro Lys Asp Thr Leu
   385                 390                 395                 400
   Met Ile Ser Arg Thr Pro Glu Val Thr Cys Val Val Val Asp Val Ser
                   405                 410                 415
   Gln Glu Asp Pro Glu Val Gln Phe Asn Trp Tyr Val Asp Gly Val Glu
               420                 425                 430
   Val His Asn Ala Lys Thr Lys Pro Arg Glu Glu Gln Phe Gln Ser Thr
           435                 440                 445
   Tyr Arg Val Val Ser Val Leu Thr Val Leu His Gln Asp Trp Leu Asn
       450                 455                 460
   Gly Lys Glu Tyr Lys Cys Lys Val Ser Asn Lys Gly Leu Pro Ser Ser
   465                 470                 475                 480
   Ile Glu Lys Thr Ile Ser Lys Ala Lys Gly Gln Pro Arg Glu Pro Gln
                   485                 490                 495
   Val Tyr Thr Leu Pro Pro Ser Gln Glu Glu Met Thr Lys Asn Gln Val
               500                 505                 510
   Ser Leu Thr Cys Leu Val Lys Gly Phe Tyr Pro Ser Asp Ile Ala Val
           515                 520                 525
   Glu Trp Glu Ser Asn Gly Gln Pro Glu Asn Asn Tyr Lys Thr Thr Pro
       530                 535                 540
   Pro Val Leu Asp Ser Asp Gly Ser Phe Phe Leu Tyr Ser Arg Leu Thr
   545                 550                 555                 560
   Val Asp Lys Ser Arg Trp Gln Glu Gly Asn Val Phe Ser Cys Ser Val
                   565                 570                 575
   Met His Glu Ala Leu His Asn His Tyr Thr Gln Lys Ser Leu Ser Leu
               580                 585                 590
   Ser Leu Gly Lys Ile Tyr Ile Trp Ala Pro Leu Ala Gly Thr Cys Gly
           595                 600                 605
   Val Leu Leu Leu Ser Leu Val Ile Thr Leu Tyr Cys Lys Arg Gly Arg
       610                 615                 620
   Lys Lys Leu Leu Tyr Ile Phe Lys Gln Pro Phe Met Arg Pro Val Gln
   625                 630                 635                 640
   Thr Thr Gln Glu Glu Asp Gly Cys Ser Cys Arg Phe Pro Glu Glu Glu
                   645                 650                 655
   Glu Gly Gly Cys Glu Leu Arg Val Lys Phe Ser Arg Ser Ala Asp Ala
               660                 665                 670
   Pro Ala Tyr Gln Gln Gly Gln Asn Gln Leu Tyr Asn Glu Leu Asn Leu
   675 680 685
   Gly Arg Arg Glu Glu Tyr Asp Val Leu Asp Lys Arg Arg Gly Arg Asp
   690 695 700
   Pro Glu Met Gly Gly Lys Pro Arg Arg Lys Asn Pro Gln Glu Gly Leu
   705 710 715 720
   Tyr Asn Glu Leu Gln Lys Asp Lys Met Ala Glu Ala Tyr Ser Glu Ile
   725 730 735
   Gly Met Lys Gly Glu Arg Arg Arg Gly Lys Gly His Asp Gly Leu Tyr
   740 745 750
   Gln Gly Leu Ser Thr Ala Thr Lys Asp Thr Tyr Asp Ala Leu His Met
   755 760 765
   Gln Ala Leu Pro Pro Arg
   770
<210> 19
   <211> 569
   <212> PRT
   <213> Artificial sequence
<220>
   <223> CD8Leader-anti-BioVL/VH-IgG4Hinge+CH3-CD8TM-41BB-CD3z
<400> 19
   Met Ala Leu Pro Val Thr Ala Leu Leu Leu Pro Leu Ala Leu Leu Leu
   1 5 10 15
   His Ala Ala Arg Pro Asp Val Val Met Thr Gln Thr Pro Leu Ser Leu
   20 25 30
   Pro Val Ser Leu Gly Asp Gln Ala Ser Ile Ser Cys Arg Ser Ser Gln
   35 40 45
   Ser Leu Val His Ser Asn Gly Asn Thr Tyr Leu His Trp Phe Leu Gln
   50 55 60
   Lys Pro Gly Gln Ser Pro Asn Leu Leu Ile Tyr Lys Val Ser Asn Arg
   65 70 75 80
   Phe Ser Gly Val Pro Asp Arg Phe Ser Gly Ser Gly Ser Gly Thr Asp
   85 90 95
   Phe Thr Leu Lys Ile Ser Arg Val Glu Ala Glu Asp Leu Gly Val Tyr
               100                 105                 110
   Phe Cys Ser Gln Ser Thr His Val Pro Tyr Thr Phe Gly Gly Gly Thr
           115                 120                 125
   Lys Leu Glu Ile Lys Gly Gly Gly Gly Ser Gly Gly Gly Gly Ser Gly
       130                 135                 140
   Gly Gly Gly Ser Glu Val Gln Leu Val Glu Ser Gly Gly Gly Leu Val
   145                 150                 155                 160
   Lys Pro Gly Gly Ser Leu Lys Leu Ser Cys Ala Val Ser Gly Phe Thr
                   165                 170                 175
   Phe Ser Asn Tyr Ala Met Ser Trp Val Arg Gln Thr Pro Glu Thr Arg
               180                 185                 190
   Leu Asn Trp Val Ala Thr Ile Thr Gly Gly Gly Ser Tyr Thr Tyr Tyr
           195                 200                 205
   Pro Asp Ser Val Lys Gly Arg Phe Thr Ile Ser Arg Asp Asn Ala Lys
       210                 215                 220
   Asp Thr Leu Tyr Leu Gln Met Ser Ser Leu Arg Ser Glu Asp Thr Ala
   225                 230                 235                 240
   Met Tyr Tyr Cys Val Arg Gln Arg Val Gly Asp Tyr Val Ser Ser Leu
                   245                 250                 255
   Leu Gly Tyr Trp Gly Gln Gly Thr Leu Val Thr Val Ser Ala Ala Ser
               260                 265                 270
   Glu Ser Lys Tyr Gly Pro Pro Cys Pro Pro Cys Pro Gly Gln Pro Arg
           275                 280                 285
   Glu Pro Gln Val Tyr Thr Leu Pro Pro Ser Gln Glu Glu Met Thr Lys
       290                 295                 300
   Asn Gln Val Ser Leu Thr Cys Leu Val Lys Gly Phe Tyr Pro Ser Asp
   305                 310                 315                 320
   Ile Ala Val Glu Trp Glu Ser Asn Gly Gln Pro Glu Asn Asn Tyr Lys
                   325                 330                 335
   Thr Thr Pro Pro Val Leu Asp Ser Asp Gly Ser Phe Phe Leu Tyr Ser
               340                 345                 350
   Arg Leu Thr Val Asp Lys Ser Arg Trp Gln Glu Gly Asn Val Phe Ser
           355                 360                 365
   Cys Ser Val Met His Glu Ala Leu His Asn His Tyr Thr Gln Lys Ser
       370                 375                 380
   Leu Ser Leu Ser Leu Gly Lys Ile Tyr Ile Trp Ala Pro Leu Ala Gly
   385                 390                 395                 400
   Thr Cys Gly Val Leu Leu Leu Ser Leu Val Ile Thr Leu Tyr Cys Lys
                   405                 410                 415
   Arg Gly Arg Lys Lys Leu Leu Tyr Ile Phe Lys Gln Pro Phe Met Arg
               420                 425                 430
   Pro Val Gln Thr Thr Gln Glu Glu Asp Gly Cys Ser Cys Arg Phe Pro
           435                 440                 445
   Glu Glu Glu Glu Gly Gly Cys Glu Leu Arg Val Lys Phe Ser Arg Ser
       450                 455                 460
   Ala Asp Ala Pro Ala Tyr Gln Gln Gly Gln Asn Gln Leu Tyr Asn Glu
   465                 470                 475                 480
   Leu Asn Leu Gly Arg Arg Glu Glu Tyr Asp Val Leu Asp Lys Arg Arg
                   485                 490                 495
   Gly Arg Asp Pro Glu Met Gly Gly Lys Pro Arg Arg Lys Asn Pro Gln
               500                 505                 510
   Glu Gly Leu Tyr Asn Glu Leu Gln Lys Asp Lys Met Ala Glu Ala Tyr
           515                 520                 525
   Ser Glu Ile Gly Met Lys Gly Glu Arg Arg Arg Gly Lys Gly His Asp
       530                 535                 540
   Gly Leu Tyr Gln Gly Leu Ser Thr Ala Thr Lys Asp Thr Tyr Asp Ala
   545                 550                 555                 560
   Leu His Met Gln Ala Leu Pro Pro Arg
                   565
<210> 20
   <211> 569
   <212> PRT
   <213> Artificial sequence
<220>
   <223> CD8Leader-anti-BioVH/VL-IgG4Hinge+CH3-CD8TM-41BB-CD3z
<400> 20
   Met Ala Leu Pro Val Thr Ala Leu Leu Leu Pro Leu Ala Leu Leu Leu
   1               5                   10                  15
   His Ala Ala Arg Pro Glu Val Gln Leu Val Glu Ser Gly Gly Gly Leu
               20                  25                  30
   Val Lys Pro Gly Gly Ser Leu Lys Leu Ser Cys Ala Val Ser Gly Phe
           35                  40                  45
   Thr Phe Ser Asn Tyr Ala Met Ser Trp Val Arg Gln Thr Pro Glu Thr
       50                  55                  60
   Arg Leu Asn Trp Val Ala Thr Ile Thr Gly Gly Gly Ser Tyr Thr Tyr
   65                  70                  75                  80
   Tyr Pro Asp Ser Val Lys Gly Arg Phe Thr Ile Ser Arg Asp Asn Ala
                   85                  90                  95
   Lys Asp Thr Leu Tyr Leu Gln Met Ser Ser Leu Arg Ser Glu Asp Thr
               100                 105                 110
   Ala Met Tyr Tyr Cys Val Arg Gln Arg Val Gly Asp Tyr Val Ser Ser
           115                 120                 125
   Leu Leu Gly Tyr Trp Gly Gln Gly Thr Leu Val Thr Val Ser Ala Gly
       130                 135                 140
   Gly Gly Gly Ser Gly Gly Gly Gly Ser Gly Gly Gly Gly Ser Asp Val
   145                 150                 155                 160
   Val Met Thr Gln Thr Pro Leu Ser Leu Pro Val Ser Leu Gly Asp Gln
                   165                 170                 175
   Ala Ser Ile Ser Cys Arg Ser Ser Gln Ser Leu Val His Ser Asn Gly
               180                 185                 190
   Asn Thr Tyr Leu His Trp Phe Leu Gln Lys Pro Gly Gln Ser Pro Asn
           195                 200                 205
   Leu Leu Ile Tyr Lys Val Ser Asn Arg Phe Ser Gly Val Pro Asp Arg
       210                 215                 220
   Phe Ser Gly Ser Gly Ser Gly Thr Asp Phe Thr Leu Lys Ile Ser Arg
   225                 230                 235                 240
   Val Glu Ala Glu Asp Leu Gly Val Tyr Phe Cys Ser Gln Ser Thr His
                   245                 250                 255
   Val Pro Tyr Thr Phe Gly Gly Gly Thr Lys Leu Glu Ile Lys Ala Ser
               260                 265                 270
   Glu Ser Lys Tyr Gly Pro Pro Cys Pro Pro Cys Pro Gly Gln Pro Arg
           275                 280                 285
   Glu Pro Gln Val Tyr Thr Leu Pro Pro Ser Gln Glu Glu Met Thr Lys
       290                 295                 300
   Asn Gln Val Ser Leu Thr Cys Leu Val Lys Gly Phe Tyr Pro Ser Asp
   305                 310                 315                 320
   Ile Ala Val Glu Trp Glu Ser Asn Gly Gln Pro Glu Asn Asn Tyr Lys
                   325                 330                 335
   Thr Thr Pro Pro Val Leu Asp Ser Asp Gly Ser Phe Phe Leu Tyr Ser
               340                 345                 350
   Arg Leu Thr Val Asp Lys Ser Arg Trp Gln Glu Gly Asn Val Phe Ser
           355                 360                 365
   Cys Ser Val Met His Glu Ala Leu His Asn His Tyr Thr Gln Lys Ser
       370                 375                 380
   Leu Ser Leu Ser Leu Gly Lys Ile Tyr Ile Trp Ala Pro Leu Ala Gly
   385                 390                 395                 400
   Thr Cys Gly Val Leu Leu Leu Ser Leu Val Ile Thr Leu Tyr Cys Lys
                   405                 410                 415
   Arg Gly Arg Lys Lys Leu Leu Tyr Ile Phe Lys Gln Pro Phe Met Arg
               420                 425                 430
   Pro Val Gln Thr Thr Gln Glu Glu Asp Gly Cys Ser Cys Arg Phe Pro
           435                 440                 445
   Glu Glu Glu Glu Gly Gly Cys Glu Leu Arg Val Lys Phe Ser Arg Ser
       450                 455                 460
   Ala Asp Ala Pro Ala Tyr Gln Gln Gly Gln Asn Gln Leu Tyr Asn Glu
   465                 470                 475                 480
   Leu Asn Leu Gly Arg Arg Glu Glu Tyr Asp Val Leu Asp Lys Arg Arg
                       485                 490                 495
   Gly Arg Asp Pro Glu Met Gly Gly Lys Pro Arg Arg Lys Asn Pro Gln
               500                 505                 510
   Glu Gly Leu Tyr Asn Glu Leu Gln Lys Asp Lys Met Ala Glu Ala Tyr
           515                 520                 525
   Ser Glu Ile Gly Met Lys Gly Glu Arg Arg Arg Gly Lys Gly His Asp
       530                 535                 540
   Gly Leu Tyr Gln Gly Leu Ser Thr Ala Thr Lys Asp Thr Tyr Asp Ala
   545                 550                 555                 560
   Leu His Met Gln Ala Leu Pro Pro Arg
                   565